# EUROPEAN PATENT SPECIFICATION

(11) **EP 2 412 828 B1**
(45) Date of publication and mention of the grant of the patent: **05.06.2013**
(21) Application number: 11184356.1
(22) Date of filing: 11.03.2008
(51) Int. Cl.: C12Q 1/68

(54) **K-ras and B-raf mutations and anti-EGFr antibody therapy**
K-RAS- und B-BAF-Mutationen und anti-EGFR-Antikörpertherapie
Mutations K-ras et B-raf et thérapie par anticorps anti-EGFr

(30) Priority: 13.03.2007 US 906976 P
(43) Date of publication of application: 01.02.2012
(62) Divisional of application: 08742064.2
(73) Proprietor: Amgen Inc., Thousand Oaks, CA 91320-1799 (US)
(72) Inventor: Siena, Salvatore, 20146 Milan (IT); Bardelli, Alberto, 10132 Torino (IT)
(74) Representative: Grünecker, Kinkeldey, Stockmair & Schwanhäusser

(56) References cited:
- WO-A-2007/001868
- WO-A1-2006/108627
- WO-A2-2006/107854
- BENVENUTI SILVIA ET AL: "Oncogenic activation of the RAS/RAF signaling pathway impairs the response of metastatic colorectal cancers to anti-epidermal growth factor receptor antibody therapies.", 15 March 2007 (2007-03-15), CANCER RESEARCH 15 MAR 2007, VOL. 67, NR. 6, PAGE(S) 2643 - 2648, XP002491315, ISSN: 0008-5472 * the whole document *
- LIÈVRE A ET AL: "KRAS mutation status is predictive of response to cetuximab therapy in colorectal cancer", CANCER RESEARCH, AMERICAN ASSOCIATION FOR CANCER RESEARCH, BALTIMORE, MD, vol. 66, no. 8, 15 April 2006 (2006-04-15) , pages 3992-3995, XP002400937, ISSN: 0008-5472
- MORONI M ET AL: "Gene copy number for epidermal growth factor receptor (EGFR) and clinical response to antiEGFR treatment in colorectal cancer: a cohort study", LANCET ONCOLOGY, LANCET PUBLISHING GROUP, LONDON, GB, vol. 6, no. 5, 1 May 2005 (2005-05-01), pages 279-286, XP004870903, ISSN: 1470-2045, DOI: 10.1016/S1470-2045(05)70102-9
- SAMOWITZ W S ET AL: "Poor Survival Associated with the BRAF V600E Mutation in Microsatellite-Stable Colon Cancers", CANCER RESEARCH, AMERICAN ASSOCIATION FOR CANCER RESEARCH, US, vol. 65, no. 14, 15 July 2005 (2005-07-15) , pages 6063-6070, XP003013060, ISSN: 0008-5472, DOI: 10.1158/0008-5472.CAN-05-0404
- CHEN MAO ET AL: "V600E mutation and resistance to anti-EGFR monoclonal antibodies in patients with metastatic colorectal cancer: a meta-analysis", MOLECULAR BIOLOGY REPORTS ; AN INTERNATIONAL JOURNAL ON MOLECULAR AND CELLULAR BIOLOGY, KLUWER ACADEMIC PUBLISHERS, DO, vol. 38, no. 4, 21 September 2010 (2010-09-21), pages 2219-2223, XP019894159, ISSN: 1573-4978, DOI: 10.1007/S11033-010-0351-4
- P. LAURENT-PUIG ET AL: "Analysis of PTEN, BRAF, and EGFR Status in Determining Benefit From Cetuximab Therapy in Wild-Type KRAS Metastatic Colon Cancer", JOURNAL OF CLINICAL ONCOLOGY, vol. 27, no. 35, 2 November 2009 (2009-11-02), pages 5924-5930, XP55013587, ISSN: 0732-183X, DOI: 10.1200/JCO.2008.21.6796
- DI NICOLANTONIO FEDERICA ET AL: "Wild-type BRAF is required for response to panitumumab or cetuximab in metastatic colorectal cancer", JOURNAL OF CLINICAL ONCOLOGY, AMERICAN SOCIETY OF CLINICAL ONCOLOGY, US, vol. 26, no. 35, 10 December 2008 (2008-12-10), pages 5705-5712, XP009123180, ISSN: 0732-183X
- DE ROOCK W ET AL: "Effects of KRAS, BRAF, NRAS, and PIK3CA mutations on the efficacy of cetuximab plus chemotherapy in chemotherapy-refractory metastatic colorectal cancer: a retrospective consortium analysis", LANCET ONCOLOGY, LANCET PUBLISHING GROUP, LONDON, GB, vol. 11, no. 8, 1 August 2010 (2010-08-01) , pages 753-762, XP027598757, ISSN: 1470-2045, DOI: 10.1016/S1470-2045(10)70130-3 [retrieved on 2010-08-01]

### SEQUENCE LISTING

<110> Amgen Inc.
<120> K-RAS AND B-RAF MUTATIONS AND ANTI-EGFR ANTIBODY THERAPY
<130> EP67067IHVSEpau
<140> not yet assigned
   <141> herewith
<150> 08 742 064.2
   <151> 2008-03-11
<150> PCT/US2008/003327
   <151> 2008-03-11
<150> 60/906,976
   <151> 2007-03-13
<160> 20
<170> PatentIn version 3.3
<210> 1
   <211> 567
   <212> DNA
   <213> Homo sapiens
<400> 1
   atgactgaat ataaacttgt ggtagttgga gctggtggcg taggcaagag tgccttgacg      60
   atacagctaa ttcagaatca ttttgtggac gaatatgatc caacaataga ggattcctac     120
   aggaagcaag tagtaattga tggagaaacc tgtctcttgg atattctcga cacagcaggt     180
   caagaggagt acagtgcaat gagggaccag tacatgagga ctggggaggg ctttctttgt     240
   gtatttgcca taaataatac taaatcattt gaagatattc accattatag agaacaaatt     300
   aaaagagtta aggactctga agatgtacct atggtcctag taggaaataa atgtgatttg     360
   ccttctagaa cagtagacac aaaacaggct caggacttag caagaagtta tggaattcct     420
   tttattgaaa catcagcaaa gacaagacag ggtgttgatg atgccttcta tacattagtt     480
   cgagaaattc gaaaacataa agaaaagatg agcaaagatg gtaaaaagaa gaaaaagaag     540
   tcaaagacaa agtgtgtaat tatgtaa                                         567
<210> 2
   <211> 188
   <212> PRT
   <213> Homo sapiens
<400> 2
<210> 3
   <211> 567
   <212> DNA
   <213> Homo sapiens
<400> 3
   atgactgaat ataaacttgt ggtagttgga gctagtggcg taggcaagag tgccttgacg      60
   atacagctaa ttcagaatca ttttgtggac gaatatgatc caacaataga ggattcctac     120
   aggaagcaag tagtaattga tggagaaacc tgtctcttgg atattctcga cacagcaggt     180
   caagaggagt acagtgcaat gagggaccag tacatgagga ctggggaggg ctttctttgt     240
   gtatttgcca taaataatac taaatcattt gaagatattc accattatag agaacaaatt     300
   aaaagagtta aggactctga agatgtacct atggtcctag taggaaataa atgtgatttg     360
   ccttctagaa cagtagacac aaaacaggct caggacttag caagaagtta tggaattcct     420
   tttattgaaa catcagcaaa gacaagacag ggtgttgatg atgccttcta tacattagtt     480
   cgagaaattc gaaaacataa agaaaagatg agcaaagatg gtaaaaagaa gaaaaagaag     540
   tcaaagacaa agtgtgtaat tatgtaa                                         567
<210> 4
   <211> 188
   <212> PRT
   <213> Homo sapiens
<400> 4
<210> 5
   <211> 567
   <212> DNA
   <213> Homo sapiens
<400> 5
   atgactgaat ataaacttgt ggtagttgga gctgttggcg taggcaagag tgccttgacg     60
   atacagctaa ttcagaatca ttttgtggac gaatatgatc caacaataga ggattcctac    120
   aggaagcaag tagtaattga tggagaaacc tgtctcttgg atattctcga cacagcaggt    180
   caagaggagt acagtgcaat gagggaccag tacatgagga ctggggaggg ctttctttgt    240
   gtatttgcca taaataatac taaatcattt gaagatattc accattatag agaacaaatt    300
   aaaagagtta aggactctga agatgtacct atggtcctag taggaaataa atgtgatttg    360
   ccttctagaa cagtagacac aaaacaggct caggacttag caagaagtta tggaattcct    420
   tttattgaaa catcagcaaa gacaagacag ggtgttgatg atgccttcta tacattagtt    480
   cgagaaattc gaaaacataa agaaaagatg agcaaagatg gtaaaaagaa gaaaaagaag    540
   tcaaagacaa agtgtgtaat tatgtaa                                        567
<210> 6
   <211> 188
   <212> PRT
   <213> Homo sapiens
<400> 6
   Met Thr Glu Tyr Lys Leu Val Val Val Gly Ala Val Gly Val Gly Lys
   1               5                   10                  15
   Ser Ala Leu Thr Ile Gln Leu Ile Gln Asn His Phe Val Asp Glu Tyr
               20                  25                  30
   Asp Pro Thr Ile Glu Asp Ser Tyr Arg Lys Gln Val Val Ile Asp Gly
           35                  40                  45
   Glu Thr Cys Leu Leu Asp Ile Leu Asp Thr Ala Gly Gln Glu Glu Tyr
       50                  55                  60
   Ser Ala Met Arg Asp Gln Tyr Met Arg Thr Gly Glu Gly Phe Leu Cys
   65                  70                  75                  80
   Val Phe Ala Ile Asn Asn Thr Lys Ser Phe Glu Asp Ile His His Tyr
                   85                  90                  95
   Arg Glu Gln Ile Lys Arg Val Lys Asp Ser Glu Asp Val Pro Met Val
               100                 105                 110
   Leu Val Gly Asn Lys Cys Asp Leu Pro Ser Arg Thr Val Asp Thr Lys
           115                 120                 125
   Gln Ala Gln Asp Leu Ala Arg Ser Tyr Gly Ile Pro Phe Ile Glu Thr
       130                 135                 140
   Ser Ala Lys Thr Arg Gln Gly Val Asp Asp Ala Phe Tyr Thr Leu Val
   145                 150                 155                 160
   Arg Glu Ile Arg Lys His Lys Glu Lys Met Ser Lys Asp Gly Lys Lys
                   165                 170                 175
   Lys Lys Lys Lys Ser Lys Thr Lys Cys Val Ile Met
               180                 185
<210> 7
   <211> 567
   <212> DNA
   <213> Homo sapiens
<400> 7
   atgactgaat ataaacttgt ggtagttgga gctgatggcg taggcaagag tgccttgacg     60
   atacagctaa ttcagaatca ttttgtggac gaatatgatc caacaataga ggattcctac    120
   aggaagcaag tagtaattga tggagaaacc tgtctcttgg atattctcga cacagcaggt    180
   caagaggagt acagtgcaat gagggaccag tacatgagga ctggggaggg ctttctttgt    240
   gtatttgcca taaataatac taaatcattt gaagatattc accattatag agaacaaatt    300
   aaaagagtta aggactctga agatgtacct atggtcctag taggaaataa atgtgatttg    360
   ccttctagaa cagtagacac aaaacaggct caggacttag caagaagtta tggaattcct    420
   tttattgaaa catcagcaaa gacaagacag ggtgttgatg atgccttcta tacattagtt    480
   cgagaaattc gaaaacataa agaaaagatg agcaaagatg gtaaaaagaa gaaaaagaag    540
   tcaaagacaa agtgtgtaat tatgtaa                                         567
<210> 8
   <211> 188
   <212> PRT
   <213> Homo sapiens
<400> 8
<210> 9
   <211> 567
   <212> DNA
   <213> Homo sapiens
<400> 9
   atgactgaat ataaacttgt ggtagttgga gctgctggcg taggcaagag tgccttgacg      60
   atacagctaa ttcagaatca ttttgtggac gaatatgatc caacaataga ggattcctac     120
   aggaagcaag tagtaattga tggagaaacc tgtctcttgg atattctcga cacagcaggt     180
   caagaggagt acagtgcaat gagggaccag tacatgagga ctggggaggg ctttctttgt     240
   gtatttgcca taaataatac taaatcattt gaagatattc accattatag agaacaaatt     300
   aaaagagtta aggactctga agatgtacct atggtcctag taggaaataa atgtgatttg     360
   ccttctagaa cagtagacac aaaacaggct caggacttag caagaagtta tggaattcct     420
   tttattgaaa catcagcaaa gacaagacag ggtgttgatg atgccttcta tacattagtt     480
   cgagaaattc gaaaacataa agaaaagatg agcaaagatg gtaaaaagaa gaaaaagaag     540
   tcaaagacaa agtgtgtaat tatgtaa                                         567
<210> 10
   <211> 188
   <212> PRT
   <213> Homo sapiens
<400> 10
<210> 11
   <211> 567
   <212> DNA
   <213> Homo sapiens
<400> 11
   atgactgaat ataaacttgt ggtagttgga gcttgtggcg taggcaagag tgccttgacg     60
   atacagctaa ttcagaatca ttttgtggac gaatatgatc caacaataga ggattcctac    120
   aggaagcaag tagtaattga tggagaaacc tgtctcttgg atattctcga cacagcaggt    180
   caagaggagt acagtgcaat gagggaccag tacatgagga ctggggaggg ctttctttgt    240
   gtatttgcca taaataatac taaatcattt gaagatattc accattatag agaacaaatt    300
   aaaagagtta aggactctga agatgtacct atggtcctag taggaaataa atgtgatttg    360
   ccttctagaa cagtagacac aaaacaggct caggacttag caagaagtta tggaattcct    420
   tttattgaaa catcagcaaa gacaagacag ggtgttgatg atgccttcta tacattagtt    480
   cgagaaattc gaaaacataa agaaaagatg agcaaagatg gtaaaaagaa gaaaaagaag    540
   tcaaagacaa agtgtgtaat tatgtaa                                        567
<210> 12
   <211> 188
   <212> PRT
   <213> Homo sapiens
<400> 12
   Met Thr Glu Tyr Lys Leu Val Val Val Gly Ala Cys Gly Val Gly Lys
   1               5                   10                  15
   Ser Ala Leu Thr Ile Gln Leu Ile Gln Asn His Phe Val Asp Glu Tyr
               20                  25                  30
   Asp Pro Thr Ile Glu Asp Ser Tyr Arg Lys Gln Val Val Ile Asp Gly
           35                  40                  45
   Glu Thr Cys Leu Leu Asp Ile Leu Asp Thr Ala Gly Gln Glu Glu Tyr
       50                  55                  60
   Ser Ala Met Arg Asp Gln Tyr Met Arg Thr Gly Glu Gly Phe Leu Cys
   65                  70                  75                  80
   Val Phe Ala Ile Asn Asn Thr Lys Ser Phe Glu Asp Ile His His Tyr
                   85                  90                  95
   Arg Glu Gln Ile Lys Arg Val Lys Asp Ser Glu Asp Val Pro Met Val
               100                 105                 110
   Leu Val Gly Asn Lys Cys Asp Leu Pro Ser Arg Thr Val Asp Thr Lys
           115                 120                 125
   Gln Ala Gln Asp Leu Ala Arg Ser Tyr Gly Ile Pro Phe Ile Glu Thr
       130                 135                 140
   Ser Ala Lys Thr Arg Gln Gly Val Asp Asp Ala Phe Tyr Thr Leu Val
   145                 150                 155                 160
   Arg Glu Ile Arg Lys His Lys Glu Lys Met Ser Lys Asp Gly Lys Lys
                   165                 170                 175
   Lys Lys Lys Lys Ser Lys Thr Lys Cys Val Ile Met
               180                 185
<210> 13
   <211> 567
   <212> DNA
   <213> Homo sapiens
<400> 13
   atgactgaat ataaacttgt ggtagttgga gctggtgccg taggcaagag tgccttgacg      60
   atacagctaa ttcagaatca ttttgtggac gaatatgatc caacaataga ggattcctac     120
   aggaagcaag tagtaattga tggagaaacc tgtctcttgg atattctcga cacagcaggt     180
   caagaggagt acagtgcaat gagggaccag tacatgagga ctggggaggg ctttctttgt     240
   gtatttgcca taaataatac taaatcattt gaagatattc accattatag agaacaaatt     300
   aaaagagtta aggactctga agatgtacct atggtcctag taggaaataa atgtgatttg     360
   ccttctagaa cagtagacac aaaacaggct caggacttag caagaagtta tggaattcct     420
   tttattgaaa catcagcaaa gacaagacag ggtgttgatg atgccttcta tacattagtt     480
   cgagaaattc gaaaacataa agaaaagatg agcaaagatg gtaaaaagaa gaaaaagaag     540
   tcaaagacaa agtgtgtaat tatgtaa                                         567
<210> 14
   <211> 188
   <212> PRT
   <213> Homo sapiens
<400> 14
   Met Thr Glu Tyr Lys Leu Val Val Val Gly Ala Gly Ala Val Gly Lys
   1               5                   10                  15
   Ser Ala Leu Thr Ile Gln Leu Ile Gln Asn His Phe Val Asp Glu Tyr
               20                  25                  30
   Asp Pro Thr Ile Glu Asp Ser Tyr Arg Lys Gln Val Val Ile Asp Gly
           35                  40                  45
   Glu Thr Cys Leu Leu Asp Ile Leu Asp Thr Ala Gly Gln Glu Glu Tyr
       50                  55                  60
   Ser Ala Met Arg Asp Gln Tyr Met Arg Thr Gly Glu Gly Phe Leu Cys
   65                  70                  75                  80
   Val Phe Ala Ile Asn Asn Thr Lys Ser Phe Glu Asp Ile His His Tyr
                   85                  90                  95
   Arg Glu Gln Ile Lys Arg Val Lys Asp Ser Glu Asp Val Pro Met Val
               100                 105                 110
   Leu Val Gly Asn Lys Cys Asp Leu Pro Ser Arg Thr Val Asp Thr Lys
           115                 120                 125
   Gln Ala Gln Asp Leu Ala Arg Ser Tyr Gly Ile Pro Phe Ile Glu Thr
       130                 135                 140
   Ser Ala Lys Thr Arg Gln Gly Val Asp Asp Ala Phe Tyr Thr Leu Val
   145                 150                 155                 160
   Arg Glu Ile Arg Lys His Lys Glu Lys Met Ser Lys Asp Gly Lys Lys
                   165                 170                 175
   Lys Lys Lys Lys Ser Lys Thr Lys Cys Val Ile Met
               180                 185
<210> 15
   <211> 567
   <212> DNA
   <213> Homo sapiens
<400> 15
   atgactgaat ataaacttgt ggtagttgga gctggtgacg taggcaagag tgccttgacg 60
   atacagctaa ttcagaatca ttttgtggac gaatatgatc caacaataga ggattcctac 120
   aggaagcaag tagtaattga tggagaaacc tgtctcttgg atattctcga cacagcaggt 180
   caagaggagt acagtgcaat gagggaccag tacatgagga ctggggaggg ctttctttgt 240
   gtatttgcca taaataatac taaatcattt gaagatattc accattatag agaacaaatt 300
   aaaagagtta aggactctga agatgtacct atggtcctag taggaaataa atgtgatttg 360
   ccttctagaa cagtagacac aaaacaggct caggacttag caagaagtta tggaattcct 420
   tttattgaaa catcagcaaa gacaagacag ggtgttgatg atgccttcta tacattagtt 480
   cgagaaattc gaaaacataa agaaaagatg agcaaagatg gtaaaaagaa gaaaaagaag 540
   tcaaagacaa agtgtgtaat tatgtaa 567
<210> 16
   <211> 188
   <212> PRT
   <213> Homo sapiens
<400> 16
<210> 17
   <211> 2301
   <212> DNA
   <213> Homo sapiens
<400> 17
   atggcggcgc tgagcggtgg cggtggtggc ggcgcggagc cgggccaggc tctgttcaac     60
   ggggacatgg agcccgaggc cggcgccggc gccggcgccg cggcctcttc ggctgcggac    120
   cctgccattc cggaggaggt gtggaatatc aaacaaatga ttaagttgac acaggaacat    180
   atagaggccc tattggacaa atttggtggg gagcataatc caccatcaat atatctggag    240
   gcctatgaag aatacaccag caagctagat gcactccaac aaagagaaca acagttattg    300
   gaatctctgg ggaacggaac tgatttttct gtttctagct ctgcatcaat ggataccgtt    360
   acatcttctt cctcttctag cctttcagtg ctaccttcat ctctttcagt ttttcaaaat    420
   cccacagatg tggcacggag caaccccaag tcaccacaaa aacctatcgt tagagtcttc    480
   ctgcccaaca aacagaggac agtggtacct gcaaggtgtg gagttacagt ccgagacagt    540
   ctaaagaaag cactgatgat gagaggtcta atcccagagt gctgtgctgt ttacagaatt    600
   caggatggag agaagaaacc aattggttgg gacactgata tttcctggct tactggagaa    660
   gaattgcatg tggaagtgtt ggagaatgtt ccacttacaa cacacaactt tgtacgaaaa    720
   acgtttttca ccttagcatt ttgtgacttt tgtcgaaagc tgcttttcca gggtttccgc    780
   tgtcaaacat gtggttataa atttcaccag cgttgtagta cagaagttcc actgatgtgt    840
   gttaattatg accaacttga tttgctgttt gtctccaagt tctttgaaca ccacccaata    900
   ccacaggaag aggcgtcctt agcagagact gccctaacat ctggatcatc cccttccgca    960
   cccgcctcgg actctattgg gccccaaatt ctcaccagtc cgtctccttc aaaatccatt   1020
   ccaattccac agcccttccg accagcagat gaagatcatc gaaatcaatt tgggcaacga   1080
   gaccgatcct catcagctcc caatgtgcat ataaacacaa tagaacctgt caatattgat   1140
   gacttgatta gagaccaagg atttcgtggt gatggaggat caaccacagg tttgtctgct   1200
   accccccctg cctcattacc tggctcacta actaacgtga aagccttaca gaaatctcca   1260
   ggacctcagc gagaaaggaa gtcatcttca tcctcagaag acaggaatcg aatgaaaaca   1320
   cttggtagac gggactcgag tgatgattgg gagattcctg atgggcagat tacagtggga   1380
   caaagaattg gatctggatc atttggaaca gtctacaagg gaaagtggca tggtgatgtg   1440
   gcagtgaaaa tgttgaatgt gacagcacct acacctcagc agttacaagc cttcaaaaat   1500
   gaagtaggag tactcaggaa aacacgacat gtgaatatcc tactcttcat gggctattcc   1560
   acaaagccac aactggctat tgttacccag tggtgtgagg gctccagctt gtatcaccat   1620
   ctccatatca ttgagaccaa atttgagatg atcaaactta tagatattgc acgacagact   1680
   gcacagggca tggattactt acacgccaag tcaatcatcc acagagacct caagagtaat   1740
   aatatatttc ttcatgaaga cctcacagta aaaataggtg attttggtct agctacagtg   1800
   aaatctcgat ggagtgggtc ccatcagttt gaacagttgt ctggatccat tttgtggatg   1860
   gcaccagaag tcatcagaat gcaagataaa aatccataca gctttcagtc agatgtatat 1920
   gcatttggaa ttgttctgta tgaattgatg actggacagt taccttattc aaacatcaac 1980
   aacagggacc agataatttt tatggtggga cgaggatacc tgtctccaga tctcagtaag 2040
   gtacggagta actgtccaaa agccatgaag agattaatgg cagagtgcct caaaaagaaa 2100
   agagatgaga gaccactctt tccccaaatt ctcgcctcta ttgagctgct ggcccgctca 2160
   ttgccaaaaa ttcaccgcag tgcatcagaa ccctccttga atcgggctgg tttccaaaca 2220
   gaggatttta gtctatatgc ttgtgcttct ccaaaaacac ccatccaggc agggggatat 2280
   ggtgcgtttc ctgtccactg a 2301
<210> 18
   <211> 766
   <212> PRT
   <213> Homo sapiens
<400> 18
<210> 19
   <211> 2301
   <212> DNA
   <213> Homo sapiens
<400> 19
   atggcggcgc tgagcggtgg cggtggtggc ggcgcggagc cgggccaggc tctgttcaac 60
   ggggacatgg agcccgaggc cggcgccggc gccggcgccg cggcctcttc ggctgcggac 120
   cctgccattc cggaggaggt gtggaatatc aaacaaatga ttaagttgac acaggaacat 180
   atagaggccc tattggacaa atttggtggg gagcataatc caccatcaat atatctggag 240
   gcctatgaag aatacaccag caagctagat gcactccaac aaagagaaca acagttattg 300
   gaatctctgg ggaacggaac tgatttttct gtttctagct ctgcatcaat ggataccgtt 360
   acatcttctt cctcttctag cctttcagtg ctaccttcat ctctttcagt ttttcaaaat 420
   cccacagatg tggcacggag caaccccaag tcaccacaaa aacctatcgt tagagtcttc 480
   ctgcccaaca aacagaggac agtggtacct gcaaggtgtg gagttacagt ccgagacagt 540
   ctaaagaaag cactgatgat gagaggtcta atcccagagt gctgtgctgt ttacagaatt 600
   caggatggag agaagaaacc aattggttgg gacactgata tttcctggct tactggagaa 660
   gaattgcatg tggaagtgtt ggagaatgtt ccacttacaa cacacaactt tgtacgaaaa 720
   acgtttttca ccttagcatt ttgtgacttt tgtcgaaagc tgcttttcca gggtttccgc 780
   tgtcaaacat gtggttataa atttcaccag cgttgtagta cagaagttcc actgatgtgt 840
   gttaattatg accaacttga tttgctgttt gtctccaagt tctttgaaca ccacccaata 900
   ccacaggaag aggcgtcctt agcagagact gccctaacat ctggatcatc cccttccgca 960
   cccgcctcgg actctattgg gccccaaatt ctcaccagtc cgtctccttc aaaatccatt 1020
   ccaattccac agcccttccg accagcagat gaagatcatc gaaatcaatt tgggcaacga 1080
   gaccgatcct catcagctcc caatgtgcat ataaacacaa tagaacctgt caatattgat 1140
   gacttgatta gagaccaagg atttcgtggt gatggaggat caaccacagg tttgtctgct 1200
   accccccctg cctcattacc tggctcacta actaacgtga aagccttaca gaaatctcca 1260
   ggacctcagc gagaaaggaa gtcatcttca tcctcagaag acaggaatcg aatgaaaaca 1320
   cttggtagac gggactcgag tgatgattgg gagattcctg atgggcagat tacagtggga 1380
   caaagaattg gatctggatc atttggaaca gtctacaagg gaaagtggca tggtgatgtg 1440
   gcagtgaaaa tgttgaatgt gacagcacct acacctcagc agttacaagc cttcaaaaat 1500
   gaagtaggag tactcaggaa aacacgacat gtgaatatcc tactcttcat gggctattcc 1560
   acaaagccac aactggctat tgttacccag tggtgtgagg gctccagctt gtatcaccat 1620
   ctccatatca ttgagaccaa atttgagatg atcaaactta tagatattgc acgacagact 1680
   gcacagggca tggattactt acacgccaag tcaatcatcc acagagacct caagagtaat 1740
   aatatatttc ttcatgaaga cctcacagta aaaataggtg attttggtct agctacagag 1800
   aaatctcgat ggagtgggtc ccatcagttt gaacagttgt ctggatccat tttgtggatg 1860
   gcaccagaag tcatcagaat gcaagataaa aatccataca gctttcagtc agatgtatat 1920
   gcatttggaa ttgttctgta tgaattgatg actggacagt taccttattc aaacatcaac 1980
   aacagggacc agataatttt tatggtggga cgaggatacc tgtctccaga tctcagtaag 2040
   gtacggagta actgtccaaa agccatgaag agattaatgg cagagtgcct caaaaagaaa 2100
   agagatgaga gaccactctt tccccaaatt ctcgcctcta ttgagctgct ggcccgctca 2160
   ttgccaaaaa ttcaccgcag tgcatcagaa ccctccttga atcgggctgg tttccaaaca 2220
   gaggatttta gtctatatgc ttgtgcttct ccaaaaacac ccatccaggc agggggatat 2280
   ggtgcgtttc ctgtccactg a 2301
<210> 20
   <211> 766
   <212> PRT
   <213> Homo sapiens
<400> 20
   Met Ala Ala Leu Ser Gly Gly Gly Gly Gly Gly Ala Glu Pro Gly Gln
   1               5                   10                  15
   Ala Leu Phe Asn Gly Asp Met Glu Pro Glu Ala Gly Ala Gly Ala Gly
               20                  25                  30
   Ala Ala Ala Ser Ser Ala Ala Asp Pro Ala Ile Pro Glu Glu Val Trp
           35                  40                  45
   Asn Ile Lys Gln Met Ile Lys Leu Thr Gln Glu His Ile Glu Ala Leu
       50                  55                  60
   Leu Asp Lys Phe Gly Gly Glu His Asn Pro Pro Ser Ile Tyr Leu Glu
   65                  70                  75                  80
   Ala Tyr Glu Glu Tyr Thr Ser Lys Leu Asp Ala Leu Gln Gln Arg Glu
                   85                  90                  95
   Gln Gln Leu Leu Glu Ser Leu Gly Asn Gly Thr Asp Phe Ser Val Ser
               100                 105                 110
   Ser Ser Ala Ser Met Asp Thr Val Thr Ser Ser Ser Ser Ser Ser Leu
           115                 120                 125
   Ser Val Leu Pro Ser Ser Leu Ser Val Phe Gln Asn Pro Thr Asp Val
       130                 135                 140
   Ala Arg Ser Asn Pro Lys Ser Pro Gln Lys Pro Ile Val Arg Val Phe
   145                 150                 155                 160
   Leu Pro Asn Lys Gln Arg Thr Val Val Pro Ala Arg Cys Gly Val Thr
                   165                 170                 175
   Val Arg Asp Ser Leu Lys Lys Ala Leu Met Met Arg Gly Leu Ile Pro
               180                 185                 190
   Glu Cys Cys Ala Val Tyr Arg Ile Gln Asp Gly Glu Lys Lys Pro Ile
           195                 200                 205
   Gly Trp Asp Thr Asp Ile Ser Trp Leu Thr Gly Glu Glu Leu His Val
       210                 215                 220
   Glu Val Leu Glu Asn Val Pro Leu Thr Thr His Asn Phe Val Arg Lys
   225                 230                 235                 240
   Thr Phe Phe Thr Leu Ala Phe Cys Asp Phe Cys Arg Lys Leu Leu Phe
                   245                 250                 255
   Gln Gly Phe Arg Cys Gln Thr Cys Gly Tyr Lys Phe His Gln Arg Cys
               260                 265                 270
   Ser Thr Glu Val Pro Leu Met Cys Val Asn Tyr Asp Gln Leu Asp Leu
           275                 280                 285
   Leu Phe Val Ser Lys Phe Phe Glu His His Pro Ile Pro Gln Glu Glu
       290                 295                 300
   Ala Ser Leu Ala Glu Thr Ala Leu Thr Ser Gly Ser Ser Pro Ser Ala
   305                 310                 315                 320
   Pro Ala Ser Asp Ser Ile Gly Pro Gln Ile Leu Thr Ser Pro Ser Pro
                   325                 330                 335
   Ser Lys Ser Ile Pro Ile Pro Gln Pro Phe Arg Pro Ala Asp Glu Asp
               340                 345                 350
   His Arg Asn Gln Phe Gly Gln Arg Asp Arg Ser Ser Ser Ala Pro Asn
           355                 360                 365
   Val His Ile Asn Thr Ile Glu Pro Val Asn Ile Asp Asp Leu Ile Arg
       370                 375                 380
   Asp Gln Gly Phe Arg Gly Asp Gly Gly Ser Thr Thr Gly Leu Ser Ala
   385                 390                 395                 400
   Thr Pro Pro Ala Ser Leu Pro Gly Ser Leu Thr Asn Val Lys Ala Leu
                   405                 410                 415
   Gln Lys Ser Pro Gly Pro Gln Arg Glu Arg Lys Ser Ser Ser Ser Ser
               420                 425                 430
   Glu Asp Arg Asn Arg Met Lys Thr Leu Gly Arg Arg Asp Ser Ser Asp
           435                 440                 445
   Asp Trp Glu Ile Pro Asp Gly Gln Ile Thr Val Gly Gln Arg Ile Gly
       450                 455                 460
   Ser Gly Ser Phe Gly Thr Val Tyr Lys Gly Lys Trp His Gly Asp Val
   465                 470                 475                 480
   Ala Val Lys Met Leu Asn Val Thr Ala Pro Thr Pro Gln Gln Leu Gln
                   485                 490                 495
   Ala Phe Lys Asn Glu Val Gly Val Leu Arg Lys Thr Arg His Val Asn
               500                 505                 510
   Ile Leu Leu Phe Met Gly Tyr Ser Thr Lys Pro Gln Leu Ala Ile Val
           515                 520                 525
   Thr Gln Trp Cys Glu Gly Ser Ser Leu Tyr His His Leu His Ile Ile
       530                 535                 540
   Glu Thr Lys Phe Glu Met Ile Lys Leu Ile Asp Ile Ala Arg Gln Thr
   545                 550                 555                 560
   Ala Gln Gly Met Asp Tyr Leu His Ala Lys Ser Ile Ile His Arg Asp
                   565                 570                 575
   Leu Lys Ser Asn Asn Ile Phe Leu His Glu Asp Leu Thr Val Lys Ile
               580                 585                 590
   Gly Asp Phe Gly Leu Ala Thr Glu Lys Ser Arg Trp Ser Gly Ser His
           595                 600                 605
   Gln Phe Glu Gln Leu Ser Gly Ser Ile Leu Trp Met Ala Pro Glu Val
       610                 615                 620
   Ile Arg Met Gln Asp Lys Asn Pro Tyr Ser Phe Gln Ser Asp Val Tyr
   625                 630                 635                 640
   Ala Phe Gly Ile Val Leu Tyr Glu Leu Met Thr Gly Gln Leu Pro Tyr
                   645                 650                 655
   Ser Asn Ile Asn Asn Arg Asp Gln Ile Ile Phe Met Val Gly Arg Gly
               660                 665                 670
   Tyr Leu Ser Pro Asp Leu Ser Lys Val Arg Ser Asn Cys Pro Lys Ala
           675                 680                 685
   Met Lys Arg Leu Met Ala Glu Cys Leu Lys Lys Lys Arg Asp Glu Arg
       690                 695                 700
   Pro Leu Phe Pro Gln Ile Leu Ala Ser Ile Glu Leu Leu Ala Arg Ser
   705                 710                 715                 720
   Leu Pro Lys Ile His Arg Ser Ala Ser Glu Pro Ser Leu Asn Arg Ala
                   725                 730                 735
   Gly Phe Gln Thr Glu Asp Phe Ser Leu Tyr Ala Cys Ala Ser Pro Lys
               740                 745                 750
   Thr Pro Ile Gln Ala Gly Gly Tyr Gly Ala Phe Pro Val His
           755                 760                 765

## Description

The present application relates to K-ras mutations, to polynucleotides encoding mutant K-ras polypeptides, and to methods of identifying K-ras mutations. The present application also relates to B-raf mutations, to polynucleotides encoding mutant B-raf polypeptides, to vectors containing those polynucleotides, and to methods of identifying B-raf mutations. The present application also relates to methods of diagnosing cancer; and methods and kits for predicting the usefulness of anti-EGFr specific binding agents in the treatment of tumors.

### BACKGROUND

Certain applications of monoclonal antibodies in cancer therapy rely on the ability of the antibody to specifically deliver to the cancerous tissues cytotoxic effector functions such as immune-enhancing isotypes, toxins or drugs. An alternative approach is to utilize monoclonal antibodies to directly affect the survival of tumor cells by depriving them of essential extracellular proliferation signals, such as those mediated by growth factors through their cell receptors. One of the attractive targets in this approach is the epidermal growth factor receptor (EGFr), which binds EGF and transforming growth factor α (TGFα) (see, e.g., Ullrich et al., Cell 61:203-212, 1990; Baselga et al., Pharmacol. Ther. 64: 127-154, 1994; Mendelsohn et al., in Biologic Therapy of Cancer 607-623, Philadelphia: J.B. Lippincott Co., 1995; Fan et al., Curr. Opin. Oncol. 10: 67-73, 1998). Binding of EGF or TGFα to EGFr, a 170 kDa transmembrane cell surface glycoprotein, triggers a cascade of cellular biochemical events, including EGFr autophosphorylation and internalization, which culminates in cell proliferation (see, e.g., Ullrich et al., Cell 61:203-212, 1990).

Several observations implicate EGFr in supporting development and progression of human solid tumors. EGFr has been demonstrated to be overexpressed on many types of human solid tumors (see, e.g., Mendelsohn Cancer Cells 7:359 (1989), Mendelsohn Cancer Biology 1:339-344 (1990), Modjtahedi and Dean Int'l J. Oncology 4:277-296 (1994)). For example, EGF-r overexpression has been observed in certain lung, breast, colon, gastric, brain, bladder, head and neck, ovarian, and prostate carcinomas (see, e.g., Modjtahedi and Dean Int'l J. Oncology 4:277-296 (1994)). The increase in receptor levels has been reported to be associated with a poor clinical prognosis (see, e.g., Baselga et al. Pharmacol. Ther. 64: 127-154, 1994; Mendelsohn et al., Bioloaic Therapy of Cancer pp. 607-623, Philadelphia: J.B. Lippincott Co., 1995; Modjtahedi et al., Intl. J. of Oncology 4:277-296, 1994; Gullick, Br. Medical Bulletin, 47:87-98, 1991; Salomon et al., Crit. Rev. Oncol. Hematol. 19: 183-232, 1995). Both epidermal growth factor (EGF) and transforming growth factor-alpha (TGF-α) have been demonstrated to bind to EGF-r and to lead to cellular proliferation and tumor growth. In many cases, increased surface EGFr expression was accompanied by production of TGFα or EGF by tumor cells, suggesting the involvement of an autocrine growth control in the progression of those tumors (see, e.g., Baselga et al. Pharmacol. Ther. 64: 127-154, 1994; Mendelsohn et al., Bioloaic Therapy of Cancer pp. 607-623, Philadelphia: J.B. Lippincott Co., 1995; Modjtahedi et al., Intl. J. of Oncology 4:277-296, 1994; Salomon et al., Crit. Rev. Oncol. Hematol. 19: 183-232, 1995).

Thus, certain groups have proposed that antibodies against EGF, TGF-α, and EGF-r may be useful in the therapy of tumors expressing or overexpressing EGF-r (see, e.g., Mendelsohn Cancer Cells 7:359 (1989), Mendelsohn Cancer Biology 1:339-344 (1990), Modjtahedi and Dean Int'l J. Oncology 4:277-296 (1994), Tosi et al. Int'l J. Cancer 62:643-650 (1995)). Indeed, it has been demonstrated that anti-EGF-r antibodies blocking EGF and TGF-α binding to the receptor appear to inhibit tumor cell proliferation. At the same time, however, anti-EGF-r antibodies have not appeared to inhibit EGF and TGF-α independent cell growth (Modjtahedi and Dean Int'l J. Oncology 4:277-296 (1994)).

Monoclonal antibodies specific to the human EGFr, capable of neutralizing EGF and TGFα binding to tumor cells and of inhibiting ligand-mediated cell proliferation in vitro, have been generated from mice and rats (see, e.g., Baselga et al., Pharmacol. Ther. 64: 127-154, 1994; Mendelsohn et al., in Bioloaic Therapy of Cancer pp. 607-623, Philadelphia: J.B. Lippincott Co., 1995; Fan et al., Curr. Opin. Oncol. 10: 67-73, 1998; Modjtahedi et al., Intl. J. Oncology 4: 277-296, 1994). Some of those antibodies, such as the mouse 108, 225 (see, e.g., Aboud-Pirak et al., J. Natl. Cancer Inst. 80: 1605-1611, 1988) and 528 (see, e.g., Baselga et al., Pharmacol. Ther. 64: 127-154, 1994; Mendelsohn et al., in Bioloaic Therapy of Cancer pp. 607-623, Philadelphia: J.B. Lippincott Co., 1995) or the rat ICR16, ICR62 and ICR64 (see, e.g., Modjtajedi et al., Intl. J. Oncology 4: 277-296, 1994; Modjtahedi et al., Br. J. Cancer 67:247-253, 1993*;* Modjtahedi et al., Br. J. Cancer 67: 254-261, 1993) monoclonal antibodies, were evaluated extensively for their ability to affect tumor growth in xenograft mouse models. Most of the anti-EGFr monoclonal antibodies were efficacious in preventing tumor formation in athymic mice when administered together with the human tumor cells (Baselga et al. Pharmacol. Ther. 64: 127-154, 1994; Modjtahedi et al., Br. J. Cancer 67: 254-261, 1993). When injected into mice bearing established human tumor xenografts, the mouse monoclonal antibodies 225 and 528 caused partial tumor regression and required the co-administration of chemotherapeutic agents, such as doxorubicin or cisplatin, for eradication of the tumors (Baselga et al. Pharmacol. Ther. 64: 127-154, 1994; Mendelsohn et al., in Bioloaic Therapy of Cancer pp. 607-623, Philadelphia: J.B. Lippincott Co., 1995; Fan et al., Cancer Res. 53: 4637-4642, 1993; Baselga et al., J. Natl. Cancer Inst. 85: 1327-1333, 1993). A chimeric version of the 225 monoclonal antibody (C225), in which the mouse antibody variable regions are linked to human constant regions, exhibited an improved *in vivo* antitumor activity but only at high doses (see, e.g., Goldstein et al., Clinical Cancer Res. 1: 1311-1318, 1995; Prewett et al., J. Immunother. Emphasis Tumor Immunol. 19: 419-427, 1996). The rat ICR16, ICR62, and ICR64 antibodies caused regression of established tumors but not their complete eradication (Modjtahedi et al., Br. J. Cancer 67: 254-261, 1993). These results established EGFr as a promising target for antibody therapy against EGFr-expressing solid tumors and led to human clinical trials with the C225 monoclonal antibody in multiple human solid cancers (see, e.g., Baselga et al. Pharmacol. Ther. 64: 127-154, 1994; Mendelsohn et al., Bioloaic Therapy of Cancer pp. 607-623, Philadelphia: J.B. Lippincott Co., 1995; Modjtahedi et al., Intl. J. of Oncology 4:277-296, 1994).

Certain advances in the biological arts made it possible to produce a fully human anti-EGFr antibody. Using mice transgenic for human immunoglobulin genes (Xenomouse™ technology, Abgenix, Inc.), human antibodies specific for human EGFr were developed (see, e.g., Mendez, Nature Genetics, 15: 146-156, 1997; Jakobovits, Adv. Drug Deliv. Rev., 31(1-2): 33-42, 1998; Jakobovits, Expert Opin. Invest. Drugs, 7(4): 607-614, 1998; Yang et al., Crit. Rev. Oncol. Hematol. 38(1):17-23, 2001; WO98/24893; WO 98/50433). One such antibody, panitumumab, a human IgG2 monoclonal antibody with an affinity of 5 x 10⁻¹¹ M for human EGFr, has been shown to block binding of EGF to the EGFr, to block receptor signaling, and to inhibit tumor cell activation and proliferation in vitro (see, e.g., WO98/50433; U.S. Patent No. 6,235,883). Studies in athymic mice have demonstrated that panitumumab also has in vivo activity, not only preventing the formation of human epidermoid carcinoma A431 xenografts in athymic mice, but also eradicating already-established large A431 tumor xenografts (see, e.g., Yang et al., Crit. Rev. Oncol. Hematol. 38(1):17-23, 2001; Yang et al., Cancer Res. 59(6):1236-43, 1999). Panitumumab has been considered for the treatment of renal carcinoma, colorectal adenocarcinoma, prostate cancer, and non small cell squamous lung carcinoma, among other cancers (see, e.g., U.S. Patent Publication No. 2004/0033543), and clinical trials are underway with that antibody. Panitumumab has been approved by the Food & Drug Administration to treat patients with metastatic colorectal cancer.

Activation of EGFr triggers at least two signalling pathways. In certain cell types, activation of EGFr prevents apoptosis by stimulation of phosphatidylinositol 3-kinase ("PI3K"). PI3K activation triggers a molecular cascade leading to the downregulation of the central pathways controlling programmed cell death (Yao, R., Science 267:2003-2006, 1995). In certain cell types, activation of EGFr initiates the MAPK cascade through Ras/Raf. Lièvre et al. Cancer Res 66:3992-5 (2006) discloses that codon 12 and 13 mutations of K-RAS are predictors of CRC resistance to treatment with cetuximab.

### SUMMARY

The invention provides methods as defined in the appended claims.

In certain embodiments, a method of predicting whether a patient will be nonresponsive to treatment with a specific binding agent to an EGFr polypeptide is disclosed. In certain embodiments, such a method comprises determining the presence or absence of a K-ras mutation in a tumor of the patient, wherein the K-ras mutation is in codon 12 or codon 13. In certain embodiments, if a K-ras mutation is present, the patient is predicted to be nonresponsive to treatment with a specific binding agent to an EGFr polypeptide.

In certain embodiments, a method of predicting whether a tumor will be nonresponsive to treatment with a specific binding agent to an EGFr polypeptide is disclosed. In certain embodiments, such a method comprises determining the presence or absence of a K-ras mutation in a sample of said tumor, wherein the K-ras mutation is in codon 12 or codon 13. In certain embodiments, the presence of the K-ras mutation indicates that the tumor will be nonresponsive to treatment with a specific binding agent to an EGFr polypeptide.

In certain embodiments, a method of predicting whether a patient will be nonresponsive to treatment with a specific binding agent to an EGFr polypeptide is disclosed. In certain embodiments, such a method comprises determining the presence or absence of a B-raf mutation in a tumor of the patient, wherein the B-raf mutation is in codon 600. In certain embodiment, if a B-raf mutation is present, the patient is predicted to be nonresponsive to treatment with a specific binding agent to an EGFr polypeptide.

In certain embodiments, a method of predicting whether a tumor will be nonresponsive to treatment with a specific binding agent to an EGFr polypeptide is disclosed. In certain embodiments, such a method comprises determining the presence or absence of a B-raf mutation in a sample of said tumor, wherein the B-raf mutation is in codon 600. In certain embodiments, the presence of the B-raf mutation indicates that the tumor will be nonresponsive to a specific binding agent to an EGFr polypeptide.

In certain embodiments, a method of predicting whether a patient will be nonresponsive to treatment with a specific binding agent to an EGFr polypeptide is disclosed. In certain embodiments, such a method comprises determining the presence or absence of a K-ras mutation in a tumor of the patient, wherein the K-ras mutation is in codon 12 or codon 13; and determining the presence or absence of a B-raf mutation in a tumor of the patient, wherein the B-raf mutation is in codon 600. In certain embodiments, if at least one of a K-ras mutation and a B-raf mutation is present, the patient is predicted to be nonresponsive to the treatment with a specific binding agent to an EGFr polypeptide.

In certain embodiments, a method of predicting whether a tumor will be nonresponsive to treatment with a specific binding agent to an EGFr polypeptide is disclosed. In certain embodiments, such a method comprises determining the presence or absence of a K-ras mutation in a sample of said tumor, wherein the K-ras mutation is in codon 12 or codon 13; and determining the presence or absence of a B-raf mutation, wherein the B-raf mutation is in codon 600. In certain embodiments, the presence of at least one of the K-ras mutation and the B-raf mutation indicates that the tumor will be nonresponsive to treatment with a specific binding agent to an EGFr polypeptide.

### BRIEF DESCRIPTION OF THE FIGURES

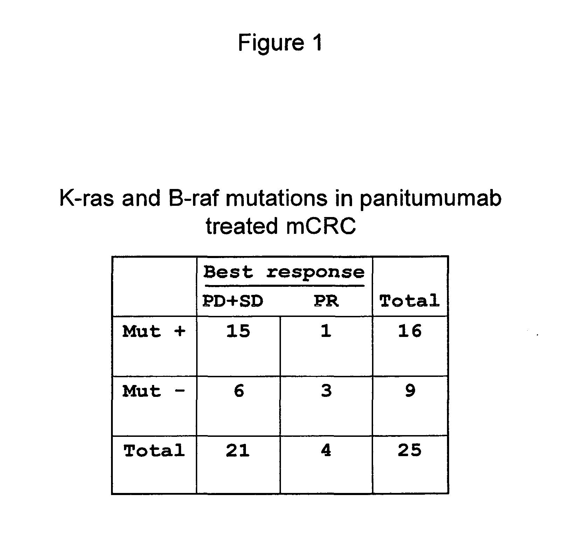

Figure 1 shows the response of patients with metastatic colorectal cancer (mCRC) treated with the antibody panitumamab. "Mut +" indicates that a patient possesses a K-ras or B-raf mutation. "Mut -" indicates that a patient does not possess a K-ras or B-raf mutation. SD stands for stable disease. PD stands for progressive disease. PR stands for partial response.

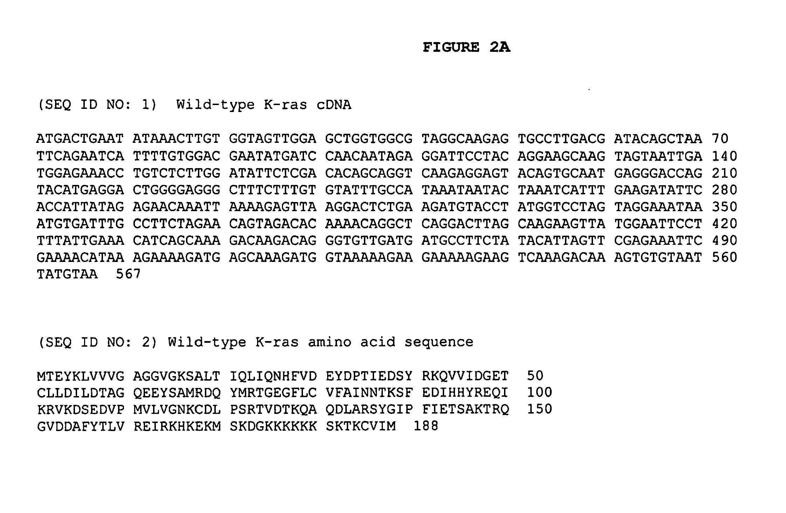

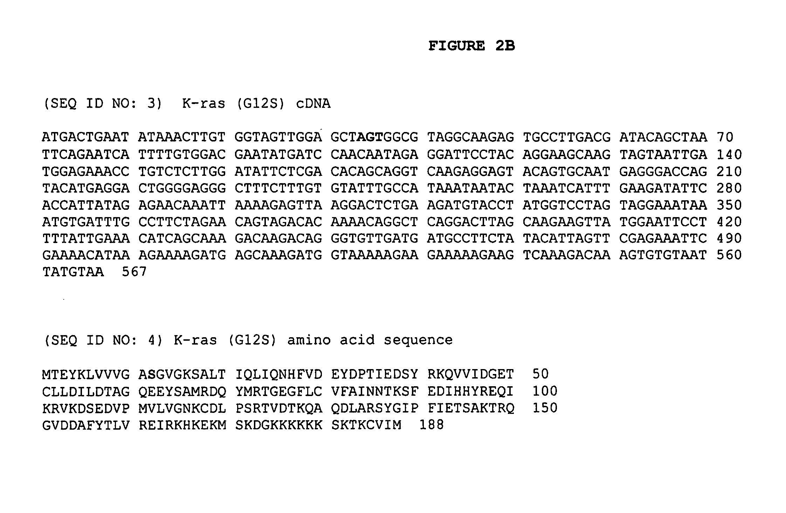

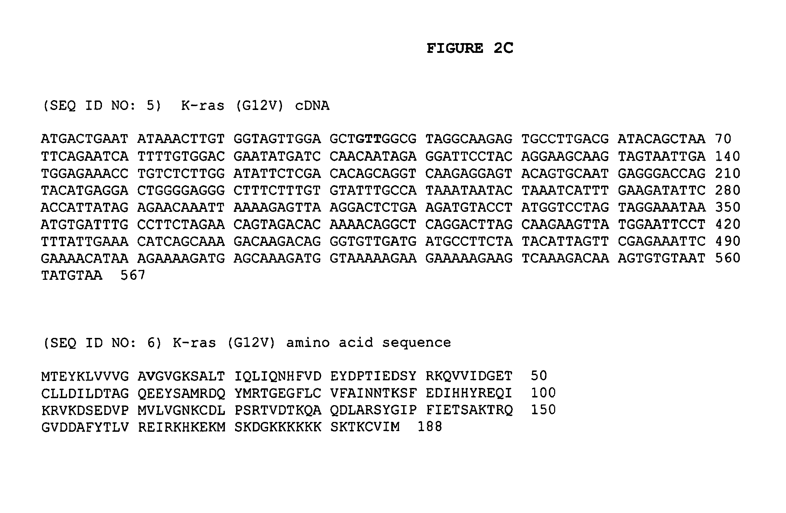

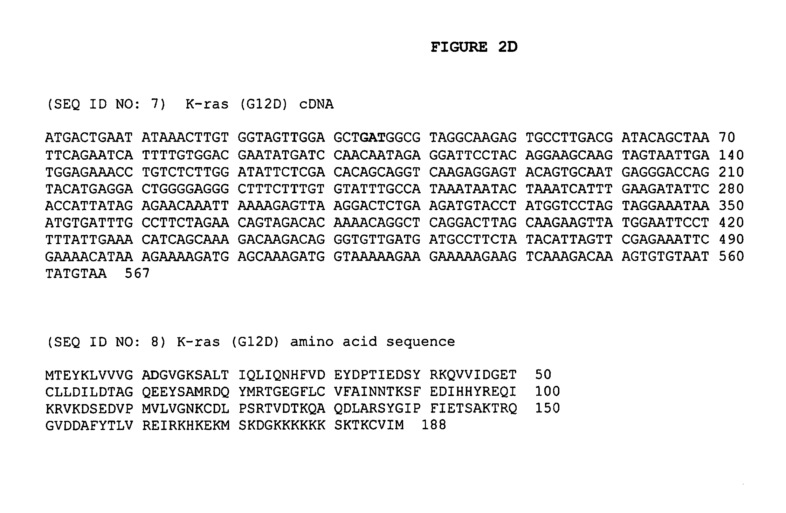

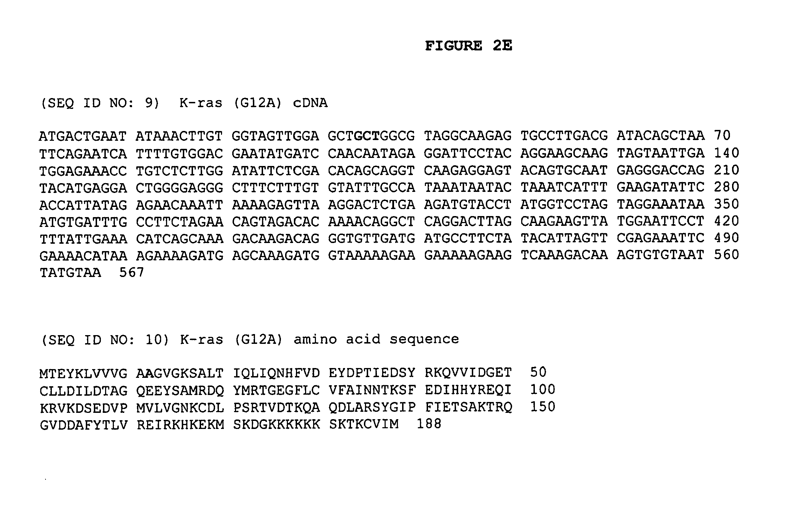

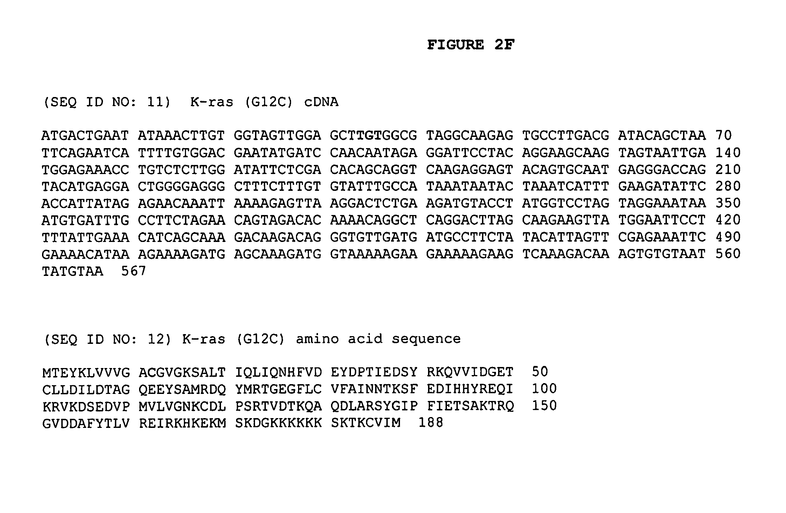

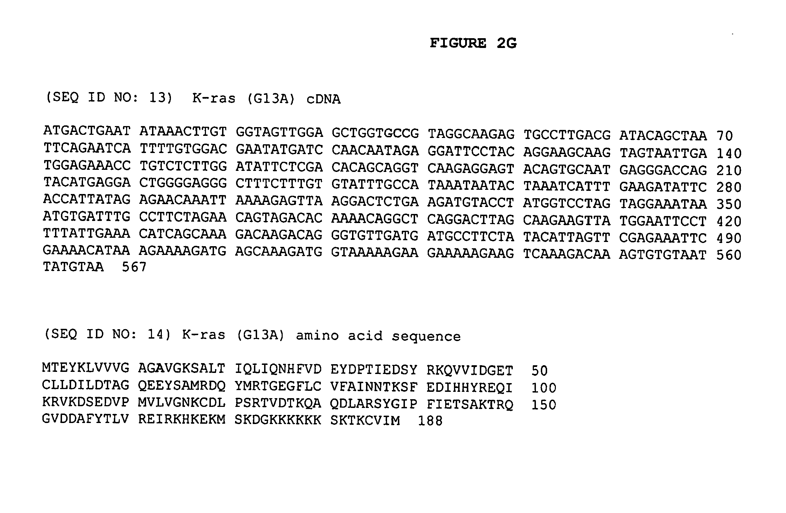

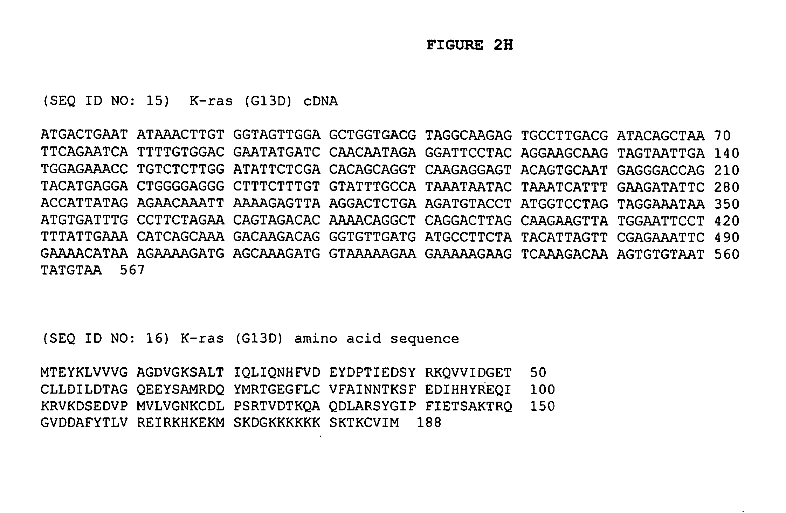

Figures 2A to 2H show the cDNA and amino acid sequences for wild-type K-ras (SEQ ID NOs: 1 and 2), G12S mutant K-ras (SEQ ID NOs: 3 and 4), G12V mutant K-ras (SEQ ID NOs: 5 and 6), G12D mutant K-ras (SEQ ID NOs: 7 and 8), G12A mutant K-ras (SEQ ID NOs: 9 and 10), G12C mutant K-ras (SEQ ID NOs: 11 and 12), G13A mutant K-ras (SEQ ID NOs: 13 and 14), and G13D mutant K-ras (SEQ ID NOs: 15 and 16).

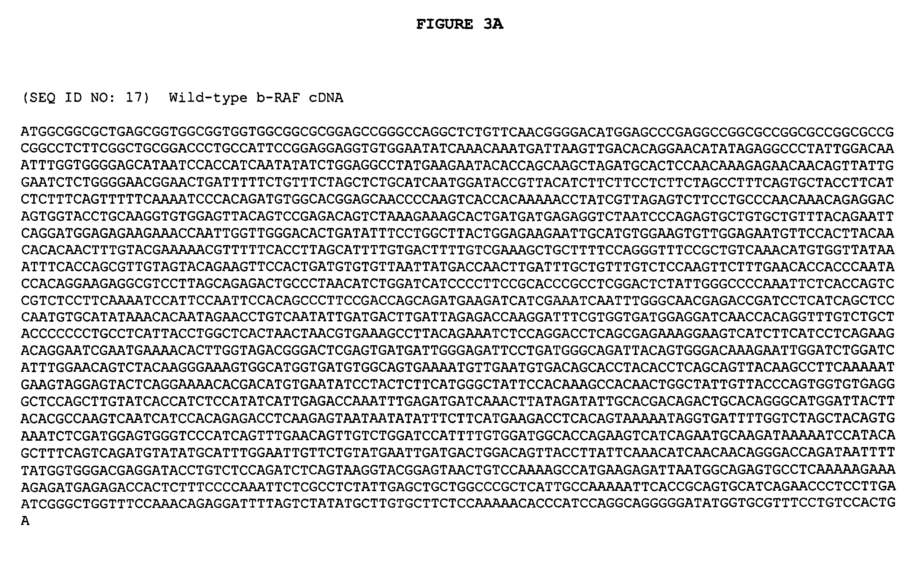

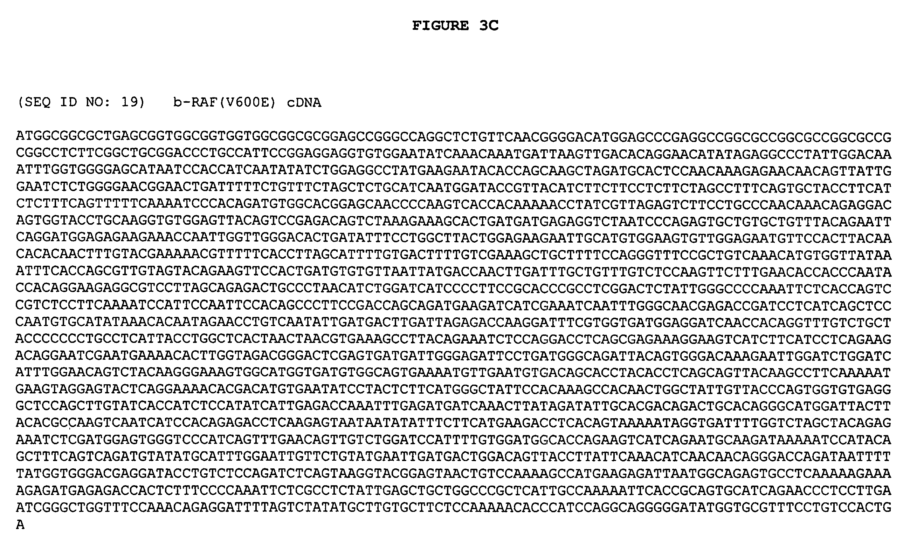

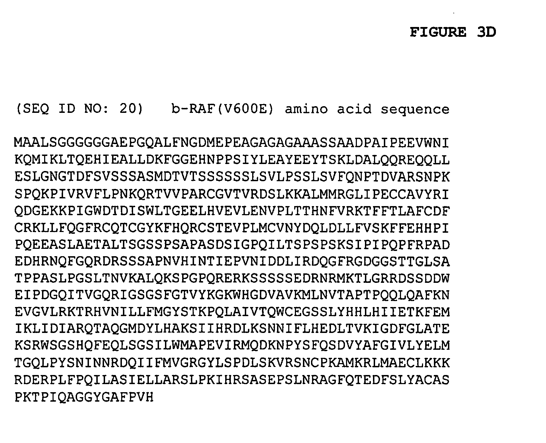

Figures 3A to 3D show the cDNA and amino acid sequences for wild-type B-raf (SEQ ID NOs: 17 and 18) and V600E mutant B-raf (SEQ ID NOs: 19 and 20).

### DETAILED DESCRIPTION OF CERTAIN EMBODIMENTS

### Definitions

Unless otherwise defined, scientific and technical terms used in connection with the present invention shall have the meanings that are commonly understood by those of ordinary skill in the art. Further, unless otherwise required by context, singular terms shall include pluralities and plural terms shall include the singular.

Generally, nomenclatures utilized in connection with, and techniques of, cell and tissue culture, molecular biology, and protein and oligo- or polynucleotide chemistry and hybridization described herein are those well known and commonly used in the art. Standard techniques are used for recombinant DNA, oligonucleotide synthesis, and tissue culture and transformation (e.g., electroporation, lipofection). Enzymatic reactions and purification techniques are performed according to the manufacturer's specifications or as commonly accomplished in the art or as described herein. The foregoing techniques and procedures are generally performed according to conventional methods well known in the art and as described in various general and more specific references that are cited and discussed throughout the present specification. *See e.g.*, Sambrook et al. Molecular Cloning: A Laboratory Manual (2d ed., Cold Spring Harbor Laboratory Press, Cold Spring Harbor, N.Y. (1989)), which is incorporated herein by reference. The nomenclatures utilized in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of patients.

In this application, the use of "or" means "and/or" unless stated otherwise. In the context of a multiple dependent claim, the use of "or" refers back to more than one preceding independent or dependent claim in the alternative only. Furthermore, the use of the term "including", as well as other forms, such as "includes" and "included", is not limiting. Also, terms such as "element" or "component" encompass both elements and components comprising one unit and elements and components that comprise more than one subunit unless specifically stated otherwise.

As utilized in accordance with the present disclosure, the following terms, unless otherwise indicated, shall be understood to have the following meanings:

The terms "isolated polynucleotide" and "isolated nucleic acid" are used interchangeably, and as used herein shall mean a polynucleotide of genomic, cDNA, or synthetic origin or some combination thereof, which by virtue of its origin (1) is not associated with all or a portion of a polynucleotide in which the "isolated polynucleotide" is found in nature, (2) is operably linked to a polynucleotide which it is not linked to in nature, or (3) does not occur in nature as part of a larger sequence.

The terms "isolated protein" and "isolated polypeptide" are used interchangeably, and as referred to herein mean a protein of cDNA, recombinant RNA, or synthetic origin, or some combination thereof, which by virtue of its origin, or source of derivation, (1) is not associated with proteins found in nature, (2) is free of other proteins from the same source, e.g. free of murine proteins, (3) is expressed by a cell from a different species, or (4) does not occur in nature.

The terms "polypeptide" and "protein" are used interchangeably and are used herein as a generic term to refer to native protein, fragments, peptides, or analogs of a polypeptide sequence. Hence, native protein, fragments, and analogs are species of the polypeptide genus.

The terminology "X#Y" in the context of a mutation in a polypeptide sequence is art-recognized, where "#" indicates the location of the mutation in terms of the amino acid number of the polypeptide, "X" indicates the amino acid found at that position in the wild-type amino acid sequence, and "Y" indicates the mutant amino acid at that position. For example, the notation "G12S" with reference to the K-ras polypeptide indicates that there is a glycine at amino acid number 12 of the wild-type K-ras sequence, and that glycine is replaced with a serine in the mutant K-ras sequence.

The terms "mutant K-ras polypeptide" and "mutant K-ras protein" are used interchangeably, and refer to a K-ras polypeptide comprising at least one K-ras mutation selected from G12S, G12V, G12D, G12A, G12C, G13A, and G13D. Certain exemplary mutant K-ras polypeptides include, but are not limited to, allelic variants, splice variants, derivative variants, substitution variants, deletion variants, and/or insertion variants, fusion polypeptides, orthologs, and interspecies homologs. In certain embodiments, a mutant K-ras polypeptide includes additional residues at the C- or N-terminus, such as, but not limited to, leader sequence residues, targeting residues, amino terminal methionine residues, lysine residues, tag residues and/or fusion protein residues.

The terms "mutant B-raf polypeptide" and "mutant B-raf protein" are used interchangeably, and refer to a B-raf polypeptide comprising V600E mutation. Certain exemplary mutant B-raf polypeptides include, but are not limited to, allelic variants, splice variants, derivative variants, substitution variants, deletion variants, and/or insertion variants, fusion polypeptides, orthologs, and interspecies homologs. In certain embodiments, a mutant B-raf polypeptide includes additional residues at the C- or N-terminus, such as, but not limited to, leader sequence residues, targeting residues, amino terminal methionine residues, lysine residues, tag residues and/or fusion protein residues.

The term "naturally-occurring" as used herein as applied to an object refers to the fact that an object can be found in nature. For example, a polypeptide or polynucleotide sequence that is present in an organism (including viruses) that can be isolated from a source in nature and which has not been intentionally modified by man in the laboratory or otherwise is naturally-occurring.

The term "operably linked" as used herein refers to the positioning of components such that they are in a relationship permitting them to function in their intended manner. A control sequence "operably linked" to a coding sequence is ligated in such a way that expression of the coding sequence is achieved under conditions compatible with the control sequences.

The term "control sequence" as used herein refers to polynucleotide sequences which are necessary to effect the expression and processing of coding sequences to which they are ligated. The nature of such control sequences differs depending upon the host organism; in prokaryotes, such control sequences generally include promoter, ribosomal binding site, and transcription termination sequences; in eukaryotes, generally, such control sequences include promoters and transcription termination sequences. The term "control sequences" is intended to include, at a minimum, all components whose presence is essential for expression and processing, and can also include additional components whose presence is advantageous, for example, leader sequences and fusion partner sequences.

The term "polynucleotide" as referred to herein means a polymeric form of nucleotides of at least 10 bases in length, either ribonucleotides or deoxynucleotides or a modified form of either type of nucleotide. The term includes single and double stranded forms of DNA.

The term "oligonucleotide" referred to herein includes naturally occurring and modified nucleotides linked together by naturally occurring, and non-naturally occurring oligonucleotide linkages. Oligonucleotides are a polynucleotide subset generally comprising a length of 200 bases or fewer. Preferably oligonucleotides are 10 to 60 bases in length and most preferably 12, 13, 14, 15, 16, 17, 18, 19, or 20 to 40 bases in length. Oligonucleotides are usually single stranded, e.g. for probes, although oligonucleotides may be double stranded, e.g. for use in the construction of a gene mutant. Oligonucleotides of the invention can be either sense or antisense oligonucleotides.

The terms "mutant K-ras polynucleotide", "mutant K-ras oligonucleotide," and "mutant K-ras nucleic acid" are used interchangeably, and refer to a polynucleotide encoding a K-ras polypeptide comprising at least one K-ras mutation selected from G12S, G12V, G12D, G12A, G12C, G13A, and G13D.

The terms "mutant B-raf polynucleotide", "mutant B-raf oligonucleotide," and "mutant B-raf nucleic acid" are used interchangeably, and refer to a polynucleotide encoding a B-raf polypeptide comprising a V600E mutation.

The term "naturally occurring nucleotides" referred to herein includes deoxyribonucleotides and ribonucleotides. The term "modified nucleotides" referred to herein includes nucleotides with modified or substituted sugar groups and the like. The term "oligonucleotide linkages" referred to herein includes oligonucleotide linkages such as phosphorothioate, phosphorodithioate, phosphoroselenoate, phosphorodiselenoate, phosphoroanilothioate, phoshoraniladate, phosphoroamidate, and the like. *See e.g.,* LaPlanche et al. Nucl. Acids Res. 14:9081 (1986); Stec et al. J. Am. Chem. Soc. 106:6077 (1984); Stein et al. Nucl. Acids Res. 16:3209 (1988); Zon et al. Anti-Cancer Drug Design 6:539 (1991); Zon et al. Oligonucleotides and Analogues: A Practical Approach, pp. 87-108 (F. Eckstein, Ed., Oxford University Press, Oxford England (1991)); Stec et al. U.S. Patent No. 5,151,510; Uhlmann and Peyman Chemical Reviews 90:543 (1990), the disclosures of which are hereby incorporated by reference. An oligonucleotide can include a label for detection, if desired.

The term "selectively hybridize" referred to herein means to detectably and specifically bind. Polynucleotides, oligonucleotides, and fragments thereof selectively hybridize to nucleic acid strands under hybridization and wash conditions that minimize appreciable amounts of detectable binding to nonspecific nucleic acids. High stringency conditions can be used to achieve selective hybridization conditions as known in the art and discussed herein. Generally, the nucleic acid sequence homology between polynucleotides, oligonucleotides, and fragments and a nucleic acid sequence of interest will be at least 80%, and more typically with preferably increasing homologies of at least 85%, 90%, 95%, 96%, 97%, 98%, 99%, and 100%. Two amino acid sequences are homologous if there is a partial or complete identity between their sequences. For example, 85% homology means that 85% of the amino acids are identical when the two sequences are aligned for maximum matching. Gaps (in either of the two sequences being matched) are allowed in maximizing matching; gap lengths of 5 or less are preferred with 2 or less being more preferred. Alternatively and preferably, two protein sequences (or polypeptide sequences derived from them of at least 30 amino acids in length) are homologous, as this term is used herein, if they have an alignment score of more than 5 (in standard deviation units) using the program ALIGN with the mutation data matrix and a gap penalty of 6 or greater. *See* Dayhoff, M.O., in Atlas of Protein Sequence and Structure, pp. 101-110 (Volume 5, National Biomedical Research Foundation (1972)) and Supplement 2 to that volume, pp. 1-10. The two sequences or parts thereof are more preferably homologous if their amino acids are greater than or equal to 50% identical when optimally aligned using the ALIGN program. The term "corresponds to" is used herein to mean that a polynucleotide sequence is homologous (i.e., is identical, not strictly evolutionarily related) to all or a portion of a reference polynucleotide sequence, or that a polypeptide sequence is identical to a reference polypeptide sequence. In contradistinction, the term "complementary to" is used herein to mean that the complementary sequence is homologous to all or a portion of a reference polynucleotide sequence. For illustration, the nucleotide sequence "TATAC" corresponds to a reference sequence "TATAC" and is complementary to a reference sequence "GTATA".

The following terms are used to describe the sequence relationships between two or more polynucleotide or amino acid sequences: "reference sequence", "comparison window", "sequence identity", "percentage of sequence identity", and "substantial identity". A "reference sequence" is a defined sequence used as a basis for a sequence comparison; a reference sequence may be a subset of a larger sequence, for example, as a segment of a full-length cDNA or gene sequence given in a sequence listing or may comprise a complete cDNA or gene sequence. Generally, a reference sequence is at least 18 nucleotides or 6 amino acids in length, or at least 24 nucleotides or 8 amino acids in length, or at least 48 nucleotides or 16 amino acids in length. Since two polynucleotides or amino acid sequences may each (1) comprise a sequence (i.e., a portion of the complete polynucleotide or amino acid sequence) that is similar between the two molecules, and (2) may further comprise a sequence that is divergent between the two polynucleotides or amino acid sequences, sequence comparisons between two (or more) molecules are typically performed by comparing sequences of the two molecules over a "comparison window" to identify and compare local regions of sequence similarity. A "comparison window", as used herein, refers to a conceptual segment of at least 18 contiguous nucleotide positions or 6 amino acids wherein a polynucleotide sequence or amino acid sequence may be compared to a reference sequence of at least 18 contiguous nucleotides or 6 amino acid sequences and wherein the portion of the polynucleotide sequence in the comparison window may comprise additions, deletions, substitutions, and the like (i.e., gaps) of 20 percent or less as compared to the reference sequence (which does not comprise additions or deletions) for optimal alignment of the two sequences. Optimal alignment of sequences for aligning a comparison window may be conducted by the local homology algorithm of Smith and Waterman Adv. Appl. Math. 2:482 (1981), by the homology alignment algorithm of Needleman and Wunsch J. Mol. Biol. 48:443 (1970), by the search for similarity method of Pearson and Lipman Proc. Natl. Acad. Sci. (U.S.A.) 85:2444 (1988), by computerized implementations of these algorithms (GAP, BESTFIT, FASTA, and TFASTA in the Wisconsin Genetics Software Package Release 7.0, (Genetics Computer Group, 575 Science Dr., Madison, Wis.), Geneworks, or MacVector software packages), or by inspection, and the best alignment (i.e., resulting in the highest percentage of homology over the comparison window) generated by the various methods is selected.

The term "sequence identity" means that two polynucleotide or amino acid sequences are identical (i.e., on a nucleotide-by-nucleotide or residue-by-residue basis) over the comparison window. The term "percentage of sequence identity" is calculated by comparing two optimally aligned sequences over the window of comparison, determining the number of positions at which the identical nucleic acid base (e.g., A, T, C, G, U, or I) or residue occurs in both sequences to yield the number of matched positions, dividing the number of matched positions by the total number of positions in the comparison window (i.e., the window size), and multiplying the result by 100 to yield the percentage of sequence identity. The terms "substantial identity" as used herein denotes a characteristic of a polynucleotide or amino acid sequence, wherein the polynucleotide or amino acid comprises a sequence that has at least 85 percent sequence identity, preferably at least 90 to 95 percent sequence identity, more usually at least 96, 97, 98, or 99 percent sequence identity as compared to a reference sequence over a comparison window of at least 18 nucleotide (6 amino acid) positions, frequently over a window of at least 24-48 nucleotide (8-16 amino acid) positions, wherein the percentage of sequence identity is calculated by comparing the reference sequence to the sequence which may include deletions or additions which total 20 percent or less of the reference sequence over the comparison window. The reference sequence may be a subset of a larger sequence.

As used herein, the twenty conventional amino acids and their abbreviations follow conventional usage. *See* Immunology - A Synthesis (2nd Edition, E.S. Golub and D.R. Gren, Eds., Sinauer Associates, Sunderland, Mass. (1991)), which is incorporated herein by reference. The term "amino acid" or "amino acid residue," as used herein, refers to naturally occurring L amino acids or to D amino acids. The commonly used one- and three-letter abbreviations for amino acids are used herein (Bruce Alberts et al., Molecular Biology of the Cell, Garland Publishing, Inc., New York (4th ed. 2002)). Stereoisomers (e.g., D-amino acids) of the twenty conventional amino acids, unnatural amino acids such as α-, α-disubstituted amino acids, N-alkyl amino acids, lactic acid, and other unconventional amino acids may also be suitable components for polypeptides of the present invention. Examples of unconventional amino acids include: 4-hydroxyproline, γ-carboxyglutamate, ε-N,N,N-trimethyllysine, ε-N-acetyllysine, O-phosphoserine, N-acetylserine, N-formylmethionine, 3-methylhistidine, 5-hydroxylysine, σ-N-methylarginine, and other similar amino acids and imino acids (e.g., 4-hydroxyproline). In the polypeptide notation used herein, the lefthand direction is the amino terminal direction and the righthand direction is the carboxy-terminal direction, in accordance with standard usage and convention.

Similarly, unless specified otherwise, the lefthand end of single-stranded polynucleotide sequences is the 5' end; the lefthand direction of double-stranded polynucleotide sequences is referred to as the 5' direction. The direction of 5' to 3' addition of nascent RNA transcripts is referred to as the transcription direction. Sequence regions on the DNA strand having the same sequence as the RNA and which are 5' to the 5' end of the RNA transcript are referred to as "upstream sequences". Sequence regions on the DNA strand having the same sequence as the RNA and which are 3' to the 3' end of the RNA transcript are referred to as "downstream sequences".

As applied to polypeptides, the term "substantial identity" means that two peptide sequences, when optimally aligned, such as by the programs GAP or BESTFIT using default gap weights, share at least 80 percent sequence identity, preferably at least 90 percent sequence identity, more preferably at least 95, 96, 97, or 98 percent sequence identity, and most preferably at least 99 percent sequence identity. Preferably, residue positions which are not identical differ by conservative amino acid substitutions. As discussed herein, minor variations in the amino acid sequences of antibodies or immunoglobulin molecules are contemplated as being encompassed by the present invention, providing that the variations in the amino acid sequence maintain at least 75%, more preferably at least 80%, 90%, 95%, and most preferably 99%. Conservative amino acid substitutions are those that take place within a family of amino acids that are related in their side chains. Genetically encoded amino acids are generally divided into families: (1) acidic=aspartate, glutamate; (2) basic=lysine, arginine, histidine; (3) nonpolar=alanine, valine, leucine, isoleucine, proline, phenylalanine, methionine, tryptophan; and (4) uncharged polar=glycine, asparagine, glutamine, cysteine, serine, threonine, tyrosine. More preferred families are: serine and threonine are aliphatic-hydroxy family; asparagine and glutamine are an amide-containing family; alanine, valine, leucine and isoleucine are an aliphatic family; phenylalanine, tryptophan, and tyrosine are an aromatic family, and cysteine and methionine as a sulfur-containing side chain family. For example, it is reasonable to expect that an isolated replacement of a leucine with an isoleucine or valine, an aspartate with a glutamate, a threonine with a serine, or a similar replacement of an amino acid with a structurally related amino acid will not have a major effect on the binding or properties of the resulting molecule, especially if the replacement does not involve an amino acid within a framework site. Preferred conservative amino acid substitution groups are: valine-leucine-isoleucine, phenylalanine-tyrosine, lysine-arginine, alanine-valine, glutamic acid-aspartic acid, cysteine-methionine, and asparagine-glutamine.

Preferred amino acid substitutions are those which: (1) reduce susceptibility to proteolysis, (2) reduce susceptibility to oxidation, (3) alter binding affinity for forming protein complexes, (4) alter binding affinities, and (5) confer or modify other physicochemical or functional properties of such analogs. Analogs can include various muteins of a sequence other than the naturally-occurring peptide sequence. For example, single or multiple amino acid substitutions (preferably conservative amino acid substitutions) may be made in the naturally-occurring sequence (preferably in the portion of the polypeptide outside the domain(s) forming intermolecular contacts. A conservative amino acid substitution should not substantially change the structural characteristics of the parent sequence (e.g., a replacement amino acid should not tend to break a helix that occurs in the parent sequence, or disrupt other types of secondary structure that characterizes the parent sequence). Examples of art-recognized polypeptide secondary and tertiary structures are described in Proteins, Structures and Molecular Principles (Creighton, Ed., W. H. Freeman and Company, New York (1984)); Introduction to Protein Structure (C. Branden and J. Tooze, eds., Garland Publishing, New York, N.Y. (1991)); and Thornton et at. Nature 354:105 (1991), which are each incorporated herein by reference.

The term "analog" as used herein refers to polypeptides which are comprised of a segment of at least 25 amino acids that has substantial identity to a portion of an amino acid sequence of a naturally occurring polypeptide and which has at least one of the activities of the naturally occurring polypeptide. Typically, polypeptide analogs comprise a conservative amino acid substitution (or addition or deletion) with respect to the naturally-occurring sequence. Analogs typically are at least 20 amino acids long, preferably at least 50 amino acids long or longer, and can often be as long as a full-length naturally-occurring polypeptide.

Peptide analogs are commonly used in the pharmaceutical industry as non-peptide drugs with properties analogous to those of the template peptide. Those types of non-peptide compound are termed "peptide mimetics" or "peptidomimetics". Fauchere, J. Adv. Drug Res. 15:29 (1986); Veber and Freidinger TINS p.392 (1985); and Evans et al. J. Med. Chem. 30:1229 (1987), which are incorporated herein by reference. Such compounds are often developed with the aid of computerized molecular modeling. Peptide mimetics that are structurally similar to therapeutically useful peptides may be used to produce an equivalent therapeutic or prophylactic effect. Generally, peptidomimetics are structurally similar to a paradigm polypeptide (i.e., a polypeptide that has a biochemical property or pharmacological activity), such as human antibody, but have one or more peptide linkages optionally replaced by a linkage selected from the group consisting of: --CH₂NH--, --CH₂S--, --CH₂-CH₂--, --CH=CH--(cis and trans),-COCH₂--, --CH(OH)CH₂--, and -CH₂SO--, by methods well known in the art. Systematic substitution of one or more amino acids of a consensus sequence with a D-amino acid of the same type (e.g., D-lysine in place of L-lysine) may be used to generate more stable peptides. In addition, constrained peptides comprising a consensus sequence or a substantially identical consensus sequence variation may be generated by methods known in the art (Rizo and Gierasch Ann. Rev. Biochem. 61:387 (1992), incorporated herein by reference); for example, by adding internal cysteine residues capable of forming intramolecular disulfide bridges which cyclize the peptide.

Preferred amino- and carboxy-termini of fragments or analogs occur near boundaries of functional domains. Structural and functional domains can be identified by comparison of the nucleotide and/or amino acid sequence data to public or proprietary sequence databases. Preferably, computerized comparison methods are used to identify sequence motifs or predicted protein conformation domains that occur in other proteins of known structure and/or function. Methods to identify protein sequences that fold into a known three-dimensional structure are known (see Bowie et al. Science 253:164 (1991)). Those of skill in the art can recognize sequence motifs and structural conformations that may be used to define structural and functional domains in accordance with the invention.

The term "specific binding agent" refers to a natural or non-natural molecule that specifically binds to a target. Examples of specific binding agents include, but are not limited to, proteins, peptides, nucleic acids, carbohydrates, lipids, and small molecule compounds. In certain embodiments, a specific binding agent is an antibody. In certain embodiments, a specific binding agent is an antigen binding region.

The term "specific binding agent to an EGFr polypeptide" refers to a specific binding agent that specifically binds any portion of an EGFr polypeptide. In certain embodiments, a specific binding agent to an EGFr polypeptide is an antibody to an EGFr polypeptide. In certain embodiments, a specific binding agent to an EGFr polypeptide is an antigen binding region. In certain embodiments, a specific binding agent to an EGFr polypeptide is an antibody to EGFr. In certain embodiments, a specific binding agent to an EGFr polypeptide is panitumumab.

The term "specific binding agent to a mutant K-ras polypeptide" refers to a specific binding agent that specifically binds any portion of a mutant K-ras polypeptide. In certain embodiments, a specific binding agent to a mutant K-ras polypeptide is an antibody to a mutant K-ras polypeptide. In certain embodiments, a specific binding agent to a mutant K-ras polypeptide is an antigen binding region.

The term "specific binding agent to a mutant B-raf polypeptide" refers to a specific binding agent that specifically binds any portion of a mutant B-raf polypeptide. In certain embodiments, a specific binding agent to a mutant B-raf polypeptide is an antibody to a mutant B-raf polypeptide. In certain embodiments, a specific binding agent to a mutant B-raf polypeptide is an antigen binding region.

The term "specifically binds" refers to the ability of a specific binding agent to bind to a target with greater affinity than it binds to a non-target. In certain embodiments, specific binding refers to binding for a target with an affinity that is at least 10, 50, 100, 250, 500, or 1000 times greater than the affinity for a non-target. In certain embodiments, affinity is determined by an affinity ELISA assay. In certain embodiments, affinity is determined by a BIAcore assay. In certain embodiments, affinity is determined by a kinetic method. In certain embodiments, affinity is determined by an equilibrium/solution method. In certain embodiments, an antibody is said to specifically bind an antigen when the dissociation constant between the antibody and one or more of its recognized epitopes is ≤1 µM, preferably ≤ 100 nM and most preferably ≤ 10 nM.

"Native antibodies and immunoglobulins", in certain instances, are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (VH) followed by a number of constant domains. Each light chain has a variable domain at one end (VL) and a constant domain at its other end; the constant domain of the light chain is aligned with the first constant domain of the heavy chain, and the light chain variable domain is aligned with the variable domain of the heavy chain. Particular amino acid residues are believed to form an interface between the light- and heavy-chain variable domains (Chothia et al. J. Mol. Biol. 186:651 (1985; Novotny and Haber, Proc. Natl. Acad. Sci. U.S.A. 82:4592 (1985); Chothia et al., Nature 342:877-883 (1989)).

The term "antibody" refers to both an intact antibody and a antigen binding fragment thereof which competes with the intact antibody for specific binding. "Antigen binding fragment thereof" refers to a portion or fragment of an intact antibody molecule, wherein the fragment retains the antigen-binding function. Binding fragments are produced by recombinant DNA techniques, or by enzymatic or chemical cleavage of intact antibodies such as by cleavage with papain. Binding fragments include Fab, Fab', F(ab')₂, Fv, single-chain antibodies ("scFv"), Fd' and Fd fragments. Methods for producing the various fragments from monoclonal antibodies are well known to those skilled in the art (see, e.g., Pluckthun, 1992, Immunol. Rev. 130:151-188). An antibody other than a "bispecific" or "bifunctional" antibody is understood to have each of its binding sites be identical. An antibody substantially inhibits adhesion of a receptor to a counterreceptor when an excess of antibody reduces the quantity of receptor bound to counterreceptor by at least about 20%, 40%, 60%, or 80%, and more usually greater than about 85%, 90%, 95%, 96%, 97%, 98%, or 99% (as measured in an in vitro competitive binding assay).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and terminal or internal amino acid sequencing by use of a spinning cup sequenator, or (2) to homogeneity by SDS-PAGE under reducing or nonreducing conditions using Coomassie blue or, preferably, silver stain. An isolated antibody includes the antibody *in situ* within recombinant cells since at least one component of the antibody's natural environment will not be present. Ordinarily, however, isolated antibody will be prepared by at least one purification step.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called complementarity-determining regions (CDRs) or hypervariable regions both in the light-chain and heavy-chain variable domains. The more highly conserved portions of variable domains are called the framework (FR). The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen-binding site of antibodies (see Kabat *et al.* (1991). The constant domains are not involved directly in binding an antibody to an antigen, but exhibit various effector functions, such as participation of the antibody in antibody-dependent cellular toxicity.

"Fv" is the minimum antibody fragment which contains a complete antigen-recognition and binding site. In a two-chain Fv species, this region comprises a dimer of one heavy- and one light-chain variable domain in tight, non-covalent association. In a single-chain Fv species, one heavy-and one light-chain variable domain can be covalently linked by a flexible peptide linker such that the light and heavy chains can associate in a "dimeric" structure analogous to that in a two-chain Fv species. It is in this configuration that the three CDRs of each variable domain interact to define an antigen-binding site on the surface of the VH-VL dimer. Collectively, the six CDRs confer antigen-binding specificity on the antibody. However, even a single variable domain (or half of an Fv comprising only three CDRs specific for an antigen) has the ability to recognize and bind antigen, although at a lower affinity than the entire binding site.

The term "hypervariable region" when used herein refers to the amino acid residues of an antibody which are responsible for antigen-binding. The hypervariable region generally comprises amino acid residues from a "complementarity determining region" or "CDR" (*e.g.* residues 24-34 (L1), 50-62 (L2), and 89-97 (L3) in the light chain variable domain and 31-55 (H1), 50-65 (H2) and 95-102 (H3) in the heavy chain variable domain; Kabat et al., Sequences of Proteins of Immunological Interest, 5th Ed. Public Health Service, National Institutes of Health, Bethesda, MD. (1991)) and/or those residues from a "hypervariable loop" (*e.g*. residues 26-32 (L1), 50-52 (L2) and 91-96 (L3) in the light chain variable domain and 26-32 ((H1), 53-55 (H2) and 96-101 (H3) in the heavy chain variable domain; Chothia and Lesk J. Mol. Biol 196:901-917 (1987)). "Framework Region" or "FR" residues are those variable domain residues other than the hypervariable region residues as herein defined.

The term "complementarity determining regions" or "CDRs," when used herein, refers to parts of immunological receptors that make contact with a specific ligand and determine its specificity. The CDRs of immunological receptors are the most variable part of the receptor protein, giving receptors their diversity, and are carried on six loops at the distal end of the receptor's variable domains, three loops coming from each of the two variable domains of the receptor.

"Antibody-dependent cell-mediated cytotoxicity" and "ADCC" refer to a cell-mediated reaction in which non-specific cytotoxic cells that express Fc receptors (FcRs) (e.g. Natural Killer (NK) cells, neutrophils, and macrophages) recognize bound antibody on a target cell and subsequently cause lysis of the target cell. The primary cells for mediating ADCC, NK cells, express FcγRIII only, whereas monocytes express FcγRI, FcγRII and FcγRIII. Fc expression on hematopoietic cells is summarized in Table 3 on page 464 of Ravetch and Kinet, Annu. Rev. Immunol 9:457-92 (1991). To assess ADCC activity of a molecule of interest, an *in vitro* ADCC assay, such as that described in US Patent No. 5,500,362, or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes et al. PNAS (USA) 95:652-656 (1988).

The term "epitope" includes any protein determinant capable of specific binding to an immunoglobulin and/or T-cell receptor. Epitopic determinants usually consist of chemically active surface groupings of molecules such as amino acids or sugar side chains and usually have specific three dimensional structural characteristics, as well as specific charge characteristics.

The term "agent" is used herein to denote a chemical compound, a mixture of chemical compounds, a biological macromolecule, or an extract made from biological materials.

As used herein, the terms "label" or "labeled" refers to incorporation of a detectable marker, e.g., by incorporation of a radiolabeled amino acid or attachment to a polypeptide of biotinyl moieties that can be detected by marked avidin (e.g., streptavidin containing a fluorescent marker or enzymatic activity that can be detected by optical or colorimetric methods). In certain situations, the label or marker can also be therapeutic. Various methods of labeling polypeptides and glycoproteins are known in the art and may be used. Examples of labels for polypeptides include, but are not limited to, the following: radioisotopes or radionuclides (*e.g*., ³H, ¹⁴C, ¹⁵N, ³⁵S, ⁹⁰Y, ⁹⁹Tc, ¹¹¹In, ¹²⁵I, ¹³¹I), fluorescent labels (*e.g*., FITC, rhodamine, lanthanide phosphors), enzymatic labels (*e.g*., horseradish peroxidase, β-galactosidase, luciferase, alkaline phosphatase), chemiluminescent groups, biotinyl groups, and predetermined polypeptide epitopes recognized by a secondary reporter (e.g., leucine zipper pair sequences, binding sites for secondary antibodies, metal binding domains, epitope tags). In some embodiments, labels are attached by spacer arms of various lengths to reduce potential steric hindrance.

The term "pharmaceutical agent or drug" as used herein refers to a chemical compound or composition capable of inducing a desired therapeutic effect when properly administered to a patient. Other chemistry terms herein are used according to conventional usage in the art, as exemplified by The McGraw-Hill Dictionary of Chemical Terms (Parker, S., Ed., McGraw-Hill, San Francisco (1985)), incorporated herein by reference).

The term "antineoplastic agent" is used herein to refer to agents that have the functional property of inhibiting a development or progression of a neoplasm in a human, particularly a malignant (cancerous) lesion, such as a carcinoma, sarcoma, lymphoma, or leukemia. Inhibition of metastasis is frequently a property of antineoplastic agents. In certain embodiments, an antineoplastic agent is panitumumab.

As used herein, "substantially pure" means an object species is the predominant species present (i.e., on a molar basis it is more abundant than any other individual species in the composition), and preferably a substantially purified fraction is a composition wherein the object species comprises at least about 50 percent (on a molar basis) of all macromolecular species present. Generally, a substantially pure composition will comprise more than about 80 percent of all macromolecular species present in the composition, more preferably more than about 85%, 90%, 95%, 96, 97, 98, or 99%. Most preferably, the object species is purified to essential homogeneity (contaminant species cannot be detected in the composition by conventional detection methods) wherein the composition consists essentially of a single macromolecular species.

The term patient includes human and animal subjects.

The terms "mammal" and "animal" for purposes of treatment refers to any animal classified as a mammal, including humans, domestic and farm animals, and zoo, sports, or pet animals, such as dogs, horses, cats, cows, etc. Preferably, the mammal is human.

The term "disease state" refers to a physiological state of a cell or of a whole mammal in which an interruption, cessation, or disorder of cellular or body functions, systems, or organs has occurred.

The terms "treat" or "treatment" refer to both therapeutic treatment and prophylactic or preventative measures, wherein the object is to prevent or slow down (lessen) an undesired physiological change or disorder, such as the development or spread of cancer. For purposes of this invention, beneficial or desired clinical results include, but are not limited to, alleviation of symptoms, diminishment of extent of disease, stabilized (*i.e*., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

The term "responsive" as used herein means that a patient or tumor shows a complete response or a partial response after administering an agent, according to RECIST (Response Evaluation Criteria in Solid Tumors). The term "nonresponsive" as used herein means that a patient or tumor shows stable disease or progressive disease after administering an agent, according to RECIST. RECIST is described, e.g., in Therasse et al., February 2000, "New Guidelines to Evaluate the Response to Treatment in Solid Tumors," J. Natl. Cancer Inst. 92(3): 205-216, which is incorporated by reference herein in its entirety. Exemplary agents include specific binding agents to an EGFr polypeptide, including but not limited to, antibodies to EGFr.

A "disorder" is any condition that would benefit from one or more treatments. This includes chronic and acute disorders or disease including those pathological conditions which predispose the mammal to the disorder in question. Non-limiting examples of disorders to be treated herein include benign and malignant tumors, leukemias, and lymphoid malignancies, in particular breast, rectal, ovarian, stomach, endometrial, salivary gland, kidney, colon, thyroid, pancreatic, prostate or bladder cancer. A preferred disorder to be treated in accordance with the present invention is a malignant tumor, such as cervical carcinomas and cervical intraepithelial squamous and glandular neoplasia, renal cell carcinoma (RCC), esophageal tumors, and carcinoma-derived cell lines.

A "disease or condition related to an EGFr polypeptide" includes one or more of the following: a disease or condition caused by an EGFr polypeptide; a disease or condition contributed to by an EGFr polypeptide; and a disease or condition that is associated with the presence of an EGFr polypeptide. In certain embodiments, a disease or condition related to an EGFr polypeptide is a cancer. Exemplary cancers include, but are not limited to, non small cell lung carcinoma, breast, colon, gastric, brain, bladder, head and neck, ovarian, and prostate carcinomas.

A "disease or condition related to a mutant K-ras polypeptide" includes one or more of the following: a disease or condition caused by a mutant K-ras polypeptide; a disease or condition contributed to by a mutant K-ras polypeptide; a disease or condition that causes a mutant K-ras polypeptide; and a disease or condition that is associated with the presence of a mutant K-ras polypeptide. In certain embodiments, the disease or condition related to a mutant K-ras polypeptide may exist in the absence of the mutant K-ras polypeptide. In certain embodiments, the disease or condition related to a mutant K-ras polypeptide may be exacerbated by the presence of a mutant K-ras polypeptide. In certain embodiments, a disease or condition related to a mutant K-ras polypeptide is a cancer. Exemplary cancers include, but are not limited to, non small cell lung carcinoma, breast, colon, gastric, brain, bladder, head and neck, ovarian, and prostate carcinomas.

A "disease or condition related to a mutant B-raf polypeptide" includes one or more of the following: a disease or condition caused by a mutant B-raf polypeptide; a disease or condition contributed to by a mutant B-raf polypeptide; a disease or condition that causes a mutant B-raf polypeptide; and a disease or condition that is associated with the presence of a mutant B-raf polypeptide. In certain embodiments, the disease or condition related to a mutant B-raf polypeptide may exist in the absence of the mutation. In certain embodiments, the disease or condition related to a mutant B-raf polypeptide may be exacerbated by the presence of a mutant B-raf polypeptide. In certain embodiments, a disease or condition related to a mutant B-raf polypeptide is a cancer. Exemplary cancers include, but are not limited to, non small cell lung carcinoma, breast, colon, gastric, brain, bladder, head and neck, ovarian, and prostate carcinomas.

In "combined therapy," patients are treated with a specific binding agent for a target antigen in combination with a chemotherapeutic or antineoplastic agent and/or radiation therapy. In certain embodiments, the specific binding agent is panitumumab. Protocol designs will address effectiveness as assessed by reduction in tumor mass as well as the ability to reduce usual doses of standard chemotherapy. These dosage reductions will allow additional and/or prolonged therapy by reducing dose-related toxicity of the chemotherapeutic agent.

"Monotherapy" refers to the treatment of a disorder by administering immunotherapy to patients without an accompanying chemotherapeutic or antineoplastic agent. In certain embodiments, monotherapy comprises administering panitumumab in the absence of a chemotherapeutic or antineoplastic agent and/or radiation therapy.

### Certain Embodiments

In certain embodiments, a method of diagnosing a disease or condition which is related to one or more K-ras mutations in a subject is provided. In certain embodiments, a method of diagnosing a disease or condition which is related to one or more B-raf mutations in a subject is disclosed.

In certain embodiments of the disclosure, a method of diagnosing a disease or condition which is related to one or more K-ras mutations in a subject comprises: (a) determining the presence or amount of expression of a mutant K-ras polypeptide in a sample from the subject; and (b) diagnosing a disease or condition which is related to one or more K-ras mutations based on the presence or amount of expression of the polypeptide. In certain embodiments of the disclosure, a method of diagnosing a disease or condition which is related to one or more K-ras mutations in a subject comprises: (a) determining the presence or amount of transcription or translation of a mutant K-ras polynucleotide in a sample from the subject; and (b) diagnosing a disease or condition which is related to one or more K-ras mutations based on the presence or amount of transcription or translation of the polynucleotide. In certain embodiments of the disclosure, the disease or condition is cancer.

In certain embodiments of the disclosure, method of diagnosing a disease or condition which is related to one or more K-ras mutations in a subject comprises: (a) determining the presence or amount of expression of a polypeptide comprising at least one amino acid sequence selected from SEQ ID NO: 4, SEQ ID NO: 6, SEQ ID NO: 8, SEQ ID NO: 10, SEQ ID NO: 12, SEQ ID NO: 14, and SEQ ID NO: 16; and (b) diagnosing a disease or condition which is related to one or more K-ras mutations based on the presence or amount of expression of the polypeptide. In certain embodiments of the disclosure, a method of diagnosing a disease or condition which is related to one or more K-ras mutations in a subject comprises: (a) determining the presence or amount of transcription or translation of a polynucleotide encoding at least one amino acid sequence selected from SEQ ID NO: 4, SEQ ID NO: 6, SEQ ID NO: 8, SEQ ID NO: 10, SEQ ID NO: 12, SEQ ID NO: 14, and SEQ ID NO: 16 in a sample from the subject; and (b) diagnosing a disease or condition which is related to one or more K-ras mutations based on the presence or amount of transcription or translation of the polynucleotide. In certain embodiments of the disclosure, the disease or condition is cancer.

In certain embodiments of the disclosure, a method of diagnosing a disease or condition which is related to one or more B-raf mutations in a subject is provided. In certain embodiments, a method of diagnosing a disease or condition which is related to one or more B-raf mutations in a subject comprises: (a) determining the presence or amount of expression of a mutant B-raf polypeptide in a sample from the subject; and (b) diagnosing a disease or condition which is related to one or more B-raf mutations based on the presence or amount of expression of the polypeptide. In certain embodiments of the disclosure, a method of diagnosing a disease or condition which is related to one or more B-raf mutations in a subject comprises: (a) determining the presence or amount of transcription or translation of a mutant B-raf polynucleotide in a sample from the subject; and (b) diagnosing a disease or condition which is related to one or more B-raf mutations based on the presence or amount of transcription or translation of the polynucleotide. In certain embodiments of the disclosure, the disease or condition is cancer.

In certain embodiments of the disclosure, a method of diagnosing a disease or condition which is related to one or more B-raf mutations in a subject comprises: (a) determining the presence or amount of expression of a polypeptide comprising the amino acid sequence of SEQ ID NO: 20 in a sample from the subject; and (b) diagnosing a disease or condition which is related to one or more B-raf mutations based on the presence or amount of expression of the polypeptide. In certain embodiments, a method of diagnosing a disease or condition which is related to one or more B-raf mutations in a subject comprises: (a) determining the presence or amount of transcription or translation of a polynucleotide encoding the amino acid sequence of SEQ ID NO: 20 in a sample from the subject; and (b) diagnosing a disease or condition which is related to one or more B-raf mutations based on the presence or amount of transcription or translation of the polynucleotide. In certain embodiments of the disclosure, the disease or condition is cancer.

In certain embodiments of the disclosure, a method of diagnosing a susceptibility to a disease or condition which is related to one or more K-ras mutations in a subject is provided. In certain embodiments, a method of diagnosing a susceptibility to a disease or condition which is related to one or more K-ras mutations in a subject comprises: (a) determining the presence or amount of expression of a mutant K-ras polypeptide in a sample from the subject; and (b) diagnosing a susceptibility to a disease or condition which is related to one or more K-ras mutations based on the presence or amount of expression of the polypeptide. In certain embodiments of the disclosure, a method of diagnosing a susceptibility to a disease or condition which is related to one or more K-ras mutations in a subject comprises: (a) determining the presence or amount of transcription or translation of a mutant K-ras polynucleotide in a sample from the subject; and (b) diagnosing a susceptibility to a disease or condition which is related to one or more K-ras mutations based on the presence or amount of transcription or translation of the polynucleotide. In certain embodiments of the disclosure, the disease or condition is cancer.

In certain embodiments of the disclosure, a method of diagnosing a susceptibility to a disease or condition which is related to one or more K-ras mutations in a subject comprises: (a) determining the presence or amount of expression of a polypeptide comprising at least one amino acid sequence selected from SEQ ID NO: 4, SEQ ID NO: 6, SEQ ID NO: 8, SEQ ID NO: 10, SEQ ID NO: 12, SEQ ID NO: 14, and SEQ ID NO: 16 in a sample from the subject; and (b) diagnosing a susceptibility to a disease or condition which is related to one or more K-ras mutations based on the presence or amount of expression of the polypeptide. In certain embodiments of the disclosure, a method of diagnosing a susceptibility to a disease or condition which is related to one or more K-ras mutations in a subject comprises: (a) determining the presence or amount of transcription or translation of a polynucleotide encoding at least one amino acid sequence selected from SEQ ID NO: 4, SEQ ID NO: 6, SEQ ID NO: 8, SEQ ID NO: 10, SEQ ID NO: 12, SEQ ID NO: 14, and SEQ ID NO: 16 in a sample from the subject; and (b) diagnosing a susceptibility to a disease or condition which is related to one or more K-ras mutations based on the presence or amount of transcription or translation of the polypeptide. In certain embodiments of the disclosure, the disease or condition is cancer.

In certain embodiments of the disclosure, a method of diagnosing a susceptibility to a disease or condition which is related to one or more B-raf mutations in a subject is provided. In certain embodiments of the disclosure, a method of diagnosing a susceptibility to a disease or condition which is related to one or more B-raf mutations in a subject comprises: (a) determining the presence or amount of expression of a mutant B-raf polypeptide in a sample from the subject; and (b) diagnosing a susceptibility to a disease or condition which is related to one or more B-raf mutations based on the presence or amount of expression of the polypeptide. In certain embodiments of the disclosure, a method of diagnosing a susceptibility to a disease or condition which is related to one or more B-raf mutations in a subject comprises: (a) determining the presence or amount of transcription or translation of a mutant B-raf polynucleotide in a sample from the subject; and (b) diagnosing a susceptibility to a disease or condition which is related to one or more B-raf mutations based on the presence or amount of transcription or translation of the polynucleotide. In certain embodiments of the disclosure, the disease or condition is cancer.

In certain embodiments of the disclosure, a method of diagnosing a susceptibility to a disease or condition which is related to one or more B-raf mutations in a subject comprises: (a) determining the presence or amount of expression of a polypeptide comprising the amino acid sequence of SEQ ID NO: 20 in a sample from the subject; and (b) diagnosing a susceptibility to a disease or condition which is related to one or more B-raf mutations based on the presence or amount of expression of the polypeptide. In certain embodiments of the disclosure, a method of diagnosing a susceptibility to a disease or condition which is related to one or more B-raf mutations in a subject comprises: (a) determining the presence or amount of transcription or translation of a polynucleotide encoding the amino acid sequence of SEQ ID NO: 20 in a sample from the subject; and (b) diagnosing a susceptibility to a disease or condition which is related to one or more B-raf mutations based on the presence or amount of transcription or translation of the polypeptide. In certain embodiments of the disclosure, the disease or condition is cancer.

In certain embodiments of the disclosure, a method of determining the presence or absence of a polynucleotide encoding a mutant K-ras polypeptide is provided. In certain embodiments of the disclosure, a method of determining the presence or absence of a polynucleotide encoding a mutant K-ras polypeptide in a sample comprises (a) exposing a sample to a probe which hybridizes to a polynucleotide encoding a region of a mutant K-ras polypeptide, wherein the region comprises at least one K-ras mutation selected from G12S, G12V, G12D, G12A, G12C, G13A, and G13D, and (b) determining the presence or absence of a polynucleotide encoding a mutant K-ras polypeptide in the sample. In certain embodiments of the disclosure, a method of determining the presence or absence of a mutant K-ras polypeptide in a sample comprises (a) exposing a sample to a probe which hybridizes to a polynucleotide encoding a region of a mutant K-ras polypeptide, wherein the region comprises at least one K-ras mutation selected from G12S, G12V, G12D, G12A, G12C, G13A, and G13D, and (b) determining the presence or absence of a mutant K-ras polypeptide in the sample.

In certain embodiments of the disclosure, a method of determining the presence or absence of a polynucleotide encoding a mutant B-raf polypeptide is provided. In certain embodiments of the disclosure, a method of determining the presence or absence of a polynucleotide encoding a mutant B-raf polypeptide in a sample comprises (a) exposing a sample to a probe which hybridizes to a polynucleotide encoding a region of a mutant B-raf polypeptide, wherein the region comprises a V600E mutation, and (b) determining the presence or absence of a polynucleotide encoding a mutant B-raf polypeptide in the sample. In certain embodiments of the disclosure, a method of determining the presence or absence of a mutant B-raf polypeptide in a sample comprises (a) exposing a sample to a probe which hybridizes to a polynucleotide encoding a region of a mutant B-raf polypeptide, wherein the region comprises a V600E mutation, and (b) determining the presence or absence of a mutant B-raf polypeptide in the sample.

In certain embodiments of the disclosure, a method for establishing a mutant K-ras population profile in a specific population of individuals is provided comprising: (a) determining the presence of at least one K-ras mutation in a genetic profile of the individuals in a population; and (b) establishing a relationship between mutant K-ras genetic profiles and the individuals. In certain such embodiments of the disclosure, the specific characteristics of the individuals include a susceptibility to developing a disease or condition which is related to a K-ras mutation. In certain such embodiments, the specific characteristics of the individuals include exhibiting a disease or condition which is related to an K-ras mutation.

In certain embodiments of the disclosure, a method of predicting nonresponsiveness to treatment with a specific binding agent to an EGFr polypeptide in a subject suffering from cancer is provided, comprising determining the presence or absence of K-ras mutation G12S in the subject. In certain such embodiments, the antibody is panitumumab.

In certain embodiments of the disclosure, a method of predicting nonresponsiveness to treatment with a specific binding agent to an EGFr polypeptide in a subject suffering from cancer is provided, comprising determining the presence or absence of K-ras mutation G12V in the subject. In certain such embodiments, the antibody is panitumumab.

In certain embodiments of the disclosure, a method of predicting nonresponsiveness to treatment with a specific binding agent to an EGFr polypeptide in a subject suffering from cancer is provided, comprising determining the presence or absence of K-ras mutation G12D in the subject. In certain such embodiments, the antibody is panitumumab.

In certain embodiments of the disclosure, a method of predicting nonresponsiveness to treatment with a specific binding agent to an EGFr polypeptide in a subject suffering from cancer is provided, comprising determining the presence or absence of K-ras mutation G12A in the subject. In certain such embodiments, the antibody is panitumumab. In certain embodiments of the disclosure, a method of predicting

In certain embodiments of the disclosure, a method of predicting nonresponsiveness to treatment with a specific binding agent to an EGFr polypeptide in a subject suffering from cancer is provided, comprising determining the presence or absence of K-ras mutation G12C in the subject. In certain such embodiments, the antibody is panitumumab.

In certain embodiments of the disclosure, a method of predicting nonresponsiveness to treatment with a specific binding agent to an EGFr polypeptide in a subject suffering from cancer is provided, comprising determining the presence or absence of K-ras mutation G13A in the subject. In certain such embodiments, the antibody is panitumumab.

In certain embodiments of the disclosure, a method of predicting nonresponsiveness to treatment with a specific binding agent to an EGFr polypeptide in a subject suffering from cancer is provided, comprising determining the presence or absence of K-ras mutation G13D in the subject. In certain such embodiments, the antibody is panitumumab.

In certain embodiments of the disclosure, a method of predicting nonresponsiveness to treatment with a specific binding agent to an EGFr polypeptide in a subject suffering from cancer is provided, comprising determining the presence or absence of a K-ras mutation at amino acid 12 of K-ras and/or amino acid 13 of K-ras in the subject. In certain such embodiments, the antibody is panitumumab.

In certain embodiments of the disclosure, a kit for detecting a polynucleotide encoding a mutant K-ras polypeptide in a subject is provided. In certain such embodiments, the kit comprises a probe which hybridizes to a polynucleotide encoding a region of a mutant K-ras polypeptide, wherein the region comprises at least one K-ras mutation selected from G12S, G12V, G12D, G12A, G12C, G13A, and G13D. In certain embodiments of the disclosure, the kit further comprises two or more amplification primers. In certain embodiments of the disclosure, the kit further comprises a detection component. In certain embodiments of the disclosure, the kit further comprises a nucleic acid sampling component.

In certain embodiments of the disclosure, a method for establishing a mutant B-raf population profile in a specific population of individuals is provided comprising: (a) determining the presence of at least one B-raf mutation in a genetic profile of the individuals in a population; and (b) establishing a relationship between mutant B-raf genetic profiles and the individuals. In certain such embodiment of the disclosure, the specific characteristics of the individuals include a susceptibility to developing a disease or condition which is related to a B-raf mutation. In certain such embodiments of the disclosure, the specific characteristics of the individuals include exhibiting a disease or condition which is related to an B-raf mutation.

In certain embodiments of the disclosure, a method of predicting nonresponsiveness to treatment with a specific binding agent to an EGFr polypeptide in a subject suffering from cancer is provided, comprising determining the presence or absence of B-raf mutation V600E in the subject. In certain such embodiments, the antibody is panitumumab.

In certain embodiments of the disclosure, a method of determining nonresponsiveness to treatment with a specific binding agent to an EGFr polypeptide in a subject suffering from cancer is provided, comprising determining the presence or absence of a B-raf mutation at amino acid 600 of B-raf in the subject. In certain such embodiments, the antibody is panitumumab.

In certain embodiments of the disclosure, a kit for detecting a polynucleotide encoding a mutant B-raf polypeptide in a subject is provided. In certain such embodiments, the kit comprises a probe which hybridizes to a polynucleotide encoding a region of a mutant B-raf polypeptide, wherein the region comprises a V600E mutation. In certain embodiments of the disclosure, the kit further comprises two or more amplification primers. In certain embodiments of the disclosure, the kit further comprises a detection component. In certain embodiments of the disclosure, the kit further comprises a nucleic acid sampling component.

In certain embodiments of the disclosure, nonresponsiveness to treatment with a specific binding agent to an EGFr polypeptide is determined using RECIST (Response Evaluation Criteria in Solid Tumors). Complete response and partial response according to RECIST are both considered to be responsive to treatment with a specific binding agent to an EGFr polypeptide. Stable disease and progressive disease are both considered to be nonresponsive to treatment with a specific binding agent to an EGFr polypeptide. RECIST is known in the art and is described, e.g., in Therasse et al., February 2000, "New Guidelines to Evaluate the Response to Treatment in Solid Tumors," J. Natl. Cancer Inst. 92(3): 205-216,

In certain embodiments, a K-ras mutation and/or a B-raf mutation is detected. In certain embodiments, a K-ras mutation and/or a B-raf mutation is detected by detecting the mutant K-ras polynucleotide and/or the mutant B-raf polynucleotide. In certain embodiments, a K-ras mutation and/or a B-raf mutation is detected by detecting the mutant K-ras polypeptide and/or the mutant B-raf polypeptide.

Certain methods of detecting a mutation in a polynucleotide are known in the art. Certain exemplary such methods include, but are not limited to, sequencing, primer extension reactions, electrophoresis, picogreen assays, oligonucleotide ligation assays, hybridization assays, TaqMan assays, SNPlex assays, and assays described, e.g., in U.S. Patent Nos. 5,470,705, 5,514,543, 5,580,732, 5,624,800, 5,807,682, 6,759,202, 6,756,204, 6,734,296, 6,395,486, and U.S. Patent Publication No. US 2003-0190646 A1.

In certain embodiments, detecting a mutation in a polynucleotide comprises first amplifying a polynucleotide that may comprise the mutation. Certain methods for amplifying a polynucleotide are known in the art. Such amplification products may be used in any of the methods described herein, or known in the art, for detecting a mutation in a polynucleotide.

Certain methods of detecting a mutation in a polypeptide are known in the art. Certain exemplary such methods include, but are not limited to, detecting using a specific binding agent specific for the mutant polypeptide. Other methods of detecting a mutant polypeptide include, but are not limited to, electrophoresis and peptide sequencing.

Certain exemplary methods of detecting a mutation in a polynucleotide and/or a polypeptide are described, e.g., in Schimanski et al. (1999) Cancer Res., 59: 5169-5175; Nagasaka et al. (2004) J. Clin. Oncol., 22: 4584-4596; PCT Publication No. WO 2007/001868 A1; U.S. Patent Publication No. 2005/0272083 A1; and Lievre et al. (2006) Cancer Res. 66: 3992-3994.

In certain embodiments, microarrays comprising one or more polynucleotides encoding one or more mutant K-ras polypeptides are provided. In certain embodiments, microarrays comprising one or more polynucleotides complementary to one or more polynucleotides encoding one or more mutant K-ras polypeptides are provided. In certain embodiments, microarrays comprising one or more polynucleotides encoding one or more mutant B-raf polypeptides are provided. In certain embodiments, microarrays comprising one or more polynucleotides complementary to one or more polynucleotides encoding one or more mutant B-raf polypeptides are provided.

In certain embodiments, the presence or absence of one or more mutant K-ras polynucleotides in two or more cell or tissue samples is assessed using microarray technology. In certain embodiments, the quantity of one or more mutant K-ras polynucleotides in two or more cell or tissue samples is assessed using microarray technology.

In certain embodiments, the presence or absence of one or more mutant B-raf polynucleotides in two or more cell or tissue samples is assessed using microarray technology. In certain embodiments, the quantity of one or more mutant B-raf polynucleotides in two or more cell or tissue samples is assessed using microarray technology.

In certain embodiments, the presence or absence of one or more mutant K-ras polypeptides in two or more cell or tissue samples is assessed using microarray technology. In certain such embodiments, mRNA is first extracted from a cell or tissue sample and is subsequently converted to cDNA, which is hybridized to the microarray. In certain such embodiments, the presence or absence of cDNA that is specifically bound to the microarray is indicative of the presence or absence of the mutant K-ras polypeptide. In certain such embodiments, the expression level of the one or more mutant K-ras polypeptides is assessed by quantitating the amount of cDNA that is specifically bound to the microarray.

In certain embodiments, the presence or absence of one or more mutant B-raf polypeptides in two or more cell or tissue samples is assessed using microarray technology. In certain such embodiments, mRNA is first extracted from a cell or tissue sample and is subsequently converted to cDNA, which is hybridized to the microarray. In certain such embodiments, the presence or absence of cDNA that is specifically bound to the microarray is indicative of the presence or absence of the mutant B-raf polypeptide. In certain such embodiments, the expression level of the one or more mutant B-raf polypeptides is assessed by quantitating the amount of cDNA that is specifically bound to the microarray.

In certain embodiments, microarrays comprising one or more specific binding agents to one or more mutant K-ras polypeptides are provided. In certain such embodiments, the presence or absence of one or more mutant K-ras polypeptides in a cell or tissue is assessed. In certain such embodiments, the quantity of one or more mutant K-ras polypeptides in a cell or tissue is assessed.

In certain embodiments, microarrays comprising one or more specific binding agents to one or more mutant B-raf polypeptides are provided. In certain such embodiments, the presence or absence of one or more mutant B-raf polypeptides in a cell or tissue is assessed. In certain such embodiments, the quantity of one or more mutant B-raf polypeptides in a cell or tissue is assessed.

The following examples, including the experiments conducted and results achieved are provided for illustrative purpose only and are not to be construed as limiting upon the claims.

### EXAMPLES

### EXAMPLE 1

### METASTATIC COLORECTAL CANCER RESPONSE TO PANITUMUMAB TREATMENT

Tumors from 25 patients with metastatic colorectal cancer were enrolled in clinical trials of panitumumab (Amgen, Thousand Oaks, CA). All patients had EGFr-expressing metastatic colorectal cancer and 1% or more malignant cells that stained for EGFr by immunohistochemical analysis with DAKO EGFRPharmDX kit (DakoCytomation, Glostrup, Denmark).

Patients received 6 mg/kg of panitumumab intravenously every 2 weeks until progression as a third-line or fourth-line treatment for patients resistant to regimens of oxaliplatin and irinotecan. Tumor response was assessed using CT or MRI and statistically analyzed using RECIST (Response Evaluation Criteria in Solid Tumors), which provides guidelines for identifying complete response, partial response, stable disease, or progressive disease based on tumor size (see, e.g., Therasse et al., February 2000, "New Guidelines to Evaluate the Response to Treatment in Solid Tumors," J. Natl. Cancer Inst. 92(3): 205-216).

Of the 25 patients, 4 showed a partial response to treatment, 8 showed stable disease, and 13 showed progressive disease, as shown in Table 1.

**Table 1: Clinical characteristics of patients with metastatic colorectal cancer treated with panitumumab.**

| **Patient ID** | **Age** | **Sex** | **Line of treatment for metastatic disease** | **Tumor response** | |
|---|---|---|---|---|---|
| | | | | **Best response** | **Duration (weeks)** |
| 1 | 59 | M | 4^{th} | PR | 31 |
| 2 | 62 | F | 3^{rd} | PR | 23 |
| 3 | 57 | M | 3^{rd} | SD | 15 |
| 4 | 78 | F | 4^{th} | PR | 24 |
| 5 | 63 | M | 3^{rd} | PR | 15 |
| 6 | 71 | M | 3^{rd} | SD | 32 |
| 7 | 60 | M | 4^{th} | SD | 24 |
| 8 | 58 | M | 4^{th} | PD | NA |
| 9 | 68 | M | 4^{th} | SD | 23 |
| 10 | 56 | M | 2^{nd} | PD | NA |
| 11 | 67 | F | 3^{rd} | PD | NA |
| 12 | 54 | M | 3^{rd} | PD | NA |
| 13 | 65 | F | 4^{th} | PD | NA |
| 14 | 57 | M | 4^{th} | PD | NA |
| 15 | 62 | F | 4^{th} | PD | NA |
| 16 | 46 | F | 3^{rd} | PD | NA |
| 17 | 53 | F | 4^{th} | PD | NA |
| 18 | 67 | M | 3^{rd} | PD | NA |
| 19 | 61 | M | 4^{th} | PD | NA |
| 20 | 70 | F | 4^{th} | PD | NA |
| 21 | 63 | F | 3^{rd} | SD | 15 |
| 22 | 44 | M | 4^{th} | SD | 16 |
| 23 | 47 | F | 3^{rd} | PD | NA |
| 24 | 52 | F | 4^{th} | SD | 16 |
| 25 | 53 | F | 4^{th} | SD | 31 |

| | | | | | |
|---|---|---|---|---|---|
| PR = partial response; SD = stable disease; PD = progressive disease | | | | | |

### EXAMPLE 2

### MUTATIONAL ANALYSIS OF K-RAS, B-RAF, AND EGFR IN PATIENTS WITH METASTATIC COLORECTAL CANCER

To determine if K-ras, B-raf, and/or EGFr mutations correlated to metastatic colorectal cancer response to panitumumab, exon 2 of K-ras, exons 15 and 21 of B-raf, and exons 9 and 20 of EGFr were sequenced from each patient.

For each patient, 10 micron paraffin-embedded samples were prepared. Two micron sections were deparaffinized, stained with hematoxylineosin and analyzed for detailes morphology. Regions displaying tumor tissue were marked and DNA extracted from the tissue as described in Moroni et al. Lancet Oncol. 6: 279-286 (2005).

Exon-specific primers and sequencing primers were designed using Primer3 software (http://frodo.wi.mit.edu/cgi-bin/primer3/primer3_www.cgi) and synthesized by Invitrogen. Exon 2 of K-ras, exons 15 and 21 of B-raf, and exons 9 and 20 of EGFr were amplified by PCR using primers specific for each exon. One skilled in the art can design appropriate primers using the gene sequences for K-ras and B-raf.

The wild-type K-ras polypeptide sequence is shown in Figure 2A (SEQ ID NO: 2; Genbank Accession No. NP_004976). The wild-type K-ras cDNA sequence is also shown in Figure 2A (SEQ ID NO: 1; Genbank Accession No. NM_004985). The genomic wild-type K-ras nucleotide sequence is found at Genbank Accession No. NM_004985.

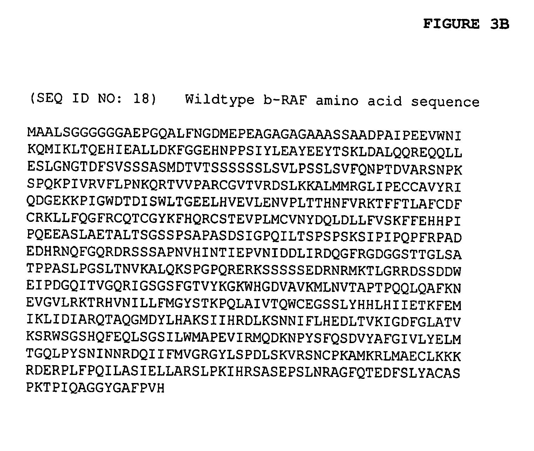

The wild-type B-raf polypeptide sequence is shown in Figure 3B (SEQ ID NO: 18; Genbank Accession No. NP_004324). The wild-type B-raf cDNA sequence is shown in Figure 3A (SEQ ID NO: 17; Genbank Accession No. NM_004333). The genomic wild-type B-raf nucleotide sequence is found, e.g., at Genbank Accession No. NT_007914.14.

The wild-type EGFr polypeptide sequence is shown, e.g. in PCT Publication No. WO 2006/091899 A1 at Figure 6C (Genbank Accession No. AAS83109). The wild-type EGFr cDNA sequence is shown, e.g. in PCT Publication No. WO 2006/091899 A1 at Figures 6A and 6B (Genbank Accession No. AC006977). The genomic wild-type EGFr nucleotide sequence is found at Genbank Accession No. AC073324.

PCR was carried out in a volume of 20 µl using a touchdown PCR program under previously-described conditions for amplifying exon-specific regions from tumor genomic DNA. See, e.g., Bardelli et al., Science 300: 949 (2003). Purified PCR products were sequenced using the BigDye® Terminator v3.1 Cycle Sequencing Kit (Applied Biosystems) and analyzed on a 3730 ABI capillary electrophoresis system. Tumor tissue from patient 13 was limited in quantity and mutations analysis was not technically possible for all exons.

The results of that analysis are shown in Table 2.

**Table 2: K-ras, B-raf, and EGFr mutational analysis of metastic colorectal cancers**

| **Patient ID** | **Sequencing analysis** | | | **Best response** |
|---|---|---|---|---|
| | **K-ras** | **B-raf** | **EGFr** | |
| 1 | WT | WT | WT | PR |
| 2 | G13D | WT | WT | PR |
| 3 | G12D | WT | WT | SD |
| 4 | WT | WT | WT | PR |
| 5 | WT | WT | WT | PR |
| 6 | G12V | WT | WT | SD |
| 7 | WT | V600E | WT | SD |
| 8 | WT | WT | WT | PD |
| 9 | WT | V600E | WT | SD |
| 10 | WT | WT | WT | PD |
| 11 | G13D | WT | WT | PD |
| 12 | WT | WT | WT | PD |
| 13 | WT | V600E | WT | PD |
| 14 | G12V | WT | WT | PD |
| 15 | WT | WT | WT | PD |
| 16 | G12V | WT | WT | PD |
| 17 | G12D | WT | WT | PD |
| 18 | WT | V600E | WT | PD |
| 19 | WT | WT | WT | PD |
| 20 | G13A | WT | WT | PD |
| 21 | G12V | WT | WT | SD |
| 22 | WT | V600E | WT | SD |
| 23 | WT | V600E | WT | PD |
| 24 | G13D | WT | WT | SD |
| 25 | WT | WT | WT | SD |

K-ras mutations were detected in 10 of the 25 tumors, or 40%. Six of those mutations were at codon 12, and 4 were at codon 13.

B-raf mutations were detected in 6 of the 25 tumors, or 24%. All of the B-raf mutations were at codon 600 (the previously described V599E mutation). No EGFr mutations were found in the 25 cancers tested.

Taken together, a total of 64% of tumors had a mutation in either K-ras or B-raf (but none of the tumors analyzed had mutations in both). Only one of the 16 tumors with either a K-ras or B-raf mutation, or 6%, showed a response to panitumumab therapy. The remaining 15 tumors with K-ras or B-raf mutations, or 94%, showed either progressive disease or stable disease after panitumumab therapy. In contrast, 3 of the 9 tumors that lacked a K-ras or B-raf mutation, or 33%, showed a reponse to panitumumab therapy.

Those data are sumarized in Figure 1. In this analysis, a mutation in K-ras codon 12 or 13 or B-raf codon 600 was correlated with nonresponsiveness to panitumumab therapy.

Other embodiments will be apparent to those skilled in the art from consideration of the specification and practice of the invention disclosed herein. It is intended that the specification and examples be considered as exemplary only.

## Claims

1. A method of predicting whether a tumor will be nonresponsive to treatment with an antibody to EGFr, comprising determining the presence or absence of a V600E B-raf mutation in a sample of said tumor; wherein the presence of the B-raf mutation indicates that the tumor will be nonresponsive to treatment with the antibody to EGFr.

2. The method of claim 1, further comprising determining the presence or absence of a K-ras mutation in a sample of said tumor, wherein the K-ras mutation is selected from G12S, G12V, G12D, G12A, G12C, G13A, and G13D; wherein the presence of the K-ras mutation indicates that the tumor will be nonresponsive to treatment with the antibody to EGFr.

3. A method of predicting whether a patient will be nonresponsive to treatment with an antibody to EGFr, comprising determining the presence or absence of a V600E B-raf mutation in a tumor sample of said patient; wherein the presence of the B-raf mutation indicates that the patient will be nonresponsive to treatment with the antibody to EGFr.

4. The method of claim 3, further comprising determining the presence or absence of a K-ras mutation in a tumor sample of said patient, wherein the K-ras mutation is selected from G12S, G12V, G12D, G12A, G12C, G13A, and G13D; wherein the presence of the K-ras mutation indicates that the patient will be nonresponsive to treatment with the antibody to EGFr.

5. The method of claim 1, 2, 3 or 4, wherein the antibody to EGFr is panitumumab.

6. The method of any one of claims 1 to 5, wherein the tumor is a colon or rectal tumor.

7. The method of any one of claims 1 to 6, wherein the tumor is obtained from a patient suffering from metastatic colon cancer.

8. The method of any one of claims 1 to 7, wherein the determining the presence or absence of the B-raf and/or K-ras mutation in a tumor or in a sample of said tumor comprises amplifying a B-raf and/or K-ras nucleic acid from the tumor or the sample of said tumor and sequencing the amplified nucleic acid.

9. The method of any one of claims 1 to 7, wherein the determining the presence or absence of the B-raf and/or K-ras mutation in a tumor or in a sample of said tumor comprises detecting a mutant B-raf and/or K-ras polypeptide in a sample of the tumor using a specific binding agent to a mutant B-raf and/or K-ras polypeptide.

## Patentansprüche

1. Verfahren zum Vorhersagen, ob ein Tumor auf eine Behandlung mit einem Antikörper gegen EGFr nicht ansprechen wird, umfassend das Bestimmen der Anwesenheit oder Abwesenheit einer V600E B-Raf-Mutation in einer Probe des Tumors, wobei die Anwesenheit der B-Raf-Mutation anzeigt, dass der Tumor auf eine Behandlung mit dem Antikörper gegen EGFr nicht ansprechen wird.

2. Verfahren nach Anspruch 1, ferner umfassend das Bestimmen der Anwesenheit oder Abwesenheit einer K-Ras-Mutation in einer Probe des Tumors, wobei die K-Ras-Mutation aus G12S, G12V, G12D, G12A, G12C, G13A und G13D ausgewählt ist; wobei die Anwesenheit der K-Ras-Mutation anzeigt, dass der Tumor auf eine Behandlung mit dem Antikörper gegen EGFr nicht ansprechen wird.

3. Verfahren zum Vorhersagen, ob ein Patient auf eine Behandlung mit einem Antikörper gegen EGFr nicht ansprechen wird, umfassend das Bestimmen der Anwesenheit oder Abwesenheit einer V600E B-Raf-Mutation in einer Tumorprobe des Patienten; wobei die Anwesenheit der B-Raf-Mutation anzeigt, dass der Patient auf eine Behandlung mit dem Antikörper gegen EGFr nicht ansprechen wird.

4. Verfahren nach Anspruch 3, ferner umfassend das Bestimmen der Anwesenheit oder Abwesenheit einer K-Ras-Mutation in einer Tumorprobe des Patienten, wobei die K-Ras-Mutation aus G12S, G12V, G12D, G12A, G12C, G13A und G13D ausgewählt ist; wobei die Anwesenheit der K-Ras-Mutation anzeigt, dass der Patient auf eine Behandlung mit dem Antikörper gegen EGFr nicht ansprechen wird.

5. Verfahren nach Anspruch 1, 2, 3 oder 4, wobei es sich bei dem Antikörper gegen EGFr um Panitumumab handelt.

6. Verfahren nach einem der Ansprüche 1 bis 5, wobei es sich bei dem Tumor um einen Kolon- oder rektalen Tumor handelt.

7. Verfahren nach einem der Ansprüche 1 bis 6, wobei der Tumor von einem Patienten erhalten wird, der an metastasierendem Kolonkrebs leidet.

8. Verfahren nach einem der Ansprüche 1 bis 7, wobei das Bestimmen der Anwesenheit oder Abwesenheit der B-Raf- und/oder K-Ras-Mutation in einem Tumor oder in einer Probe des Tumors umfasst, eine B-Raf- und/oder K-Ras-Nucleinsäure von dem Tumor oder der Probe des Tumors zu amplifizieren und die amplifizierte Nucleinsäure zu sequenzieren.

9. Verfahren nach einem der Ansprüche 1 bis 7, wobei das Bestimmen der Anwesenheit oder Abwesenheit der B-Raf- und/oder K-Ras-Mutation in einem Tumor oder in einer Probe des Tumors umfasst, ein mutantes B-Raf- und/oder K-Ras-Polypeptid in einer Probe des Tumors unter Verwendung eines spezifischen bindenden Mittels gegen ein mutantes B-Raf- und/oder K-Ras-Polypepitd nachzuweisen.

## Revendications

1. Procédé de prévision du fait qu'une tumeur sera insensible à un traitement avec un anticorps du EGFr, comprenant la détermination de la présence ou de l'absence d'une mutation V600E B-raf dans un échantillon de ladite tumeur, pour lequel la présence de la mutation B-raf indique que la tumeur sera insensible à un traitement avec l'anticorps du EGFr.

2. Procédé selon la revendication 1, comprenant en outre la détermination de la présence ou de l'absence d'une mutation K-ras dans un échantillon de ladite tumeur, pour lequel la mutation K-ras est sélectionnée parmi les G12S, G12V, G12D, G12A, G12C, G13A et G13D, pour lequel la présence de la mutation K-ras indique que la tumeur sera insensible à un traitement avec l'anticorps du EGFr.

3. Procédé de prévision du fait qu'un patient sera insensible à un traitement avec un anticorps du EGFr, comprenant la détermination de la présence ou de l'absence d'une mutation V600E B-raf dans un échantillon d'une tumeur dudit patient, pour lequel la présence de la mutation B-raf indique que le patient sera insensible à un traitement avec l'anticorps du EGFr.

4. Procédé selon la revendication 3, comprenant en outre la détermination de la présence ou de l'absence d'une mutation K-ras dans un échantillon d'une tumeur dudit patient, pour lequel la mutation K-ras est sélectionnée parmi les G12S, G12V, G12D, G12A, G12C, G13A et G13D, pour lequel la présence de la mutation K-ras indique que le patient sera insensible à un traitement avec l'anticorps du EGFr.

5. Procédé selon les revendications 1, 2, 3 ou 4, pour lequel l'anticorps du EGFr est un panitumumab.

6. Procédé selon l'une quelconque des revendications 1 à 5, pour lequel la tumeur est une tumeur du colon ou rectale.

7. Procédé selon l'une quelconque des revendications 1 à 6, pour lequel la tumeur est obtenue chez un patient soufrant d'un cancer métastatique du colon.

8. Procédé selon l'une quelconque des revendications 1 à 7, pour lequel la détermination de la présence ou de l'absence de la mutation B-raf et/ou K-ras dans une tumeur ou dans un échantillon de ladite tumeur comprend une amplification d'un acide nucléique B-raf et/ou K-ras de la tumeur ou de l'échantillon de ladite tumeur et un séquençage de l'acide nucléique amplifié.

9. Procédé selon l'une quelconque des revendications 1 à 7, pour lequel la détermination de la présence ou de l'absence de la mutation B-raf et/ou K-ras dans une tumeur ou dans un échantillon de ladite tumeur comprend la détection d'un polypeptide B-raf et/ou K-ras mutant dans un échantillon de la tumeur en utilisant un agent de liaison spécifique à un polypeptide B-raf et/ou K-ras mutant.
